# EUROPÄISCHE PATENTSCHRIFT

(11) **EP 3 503 995 B1**
(45) Veröffentlichungstag und Bekanntmachung des Hinweises auf die Patenterteilung: **21.10.2020**
(21) Anmeldenummer: 16760012.1
(22) Anmeldetag: 24.08.2016
(51) Int. Cl.: B01J 19/00, B01J 19/24

(54) **VORRICHTUNG ZUR ENERGIE-OPTIMIERTEN ERZEUGUNG VON FLUIDWIRBELN IN EINER REAKTIONSKAMMER**
DEVICE FOR THE ENERGY-OPTIMISED PRODUCTION OF FLUID EDDIES IN A REACTION CHAMBER
DISPOSITIF DE PRODUCTION OPTIMISÉE EN TERMES D'ÉNERGIE DE TURBULENCES FLUIDIQUES DANS UNE CHAMBRE RÉACTIONNELLE

(43) Veröffentlichungstag der Anmeldung: 03.07.2019
(73) Patentinhaber: Hydro Intelligence Water GmbH, 87642 Halblech (DE)
(72) Erfinder: LEHMANN, Jörg, 49808 Lingen (Ems) (DE); LINDEN, Olaf, 51427 Bergisch Gladbach (DE)
(74) Vertreter: Jeck, Anton
(86) Internationale Anmeldenummer: PCT/EP2016/069983
(87) Internationale Veröffentlichungsnummer: WO 2018/036623

(56) Entgegenhaltungen:
- EP-A1- 0 392 545
- EP-B1- 1 294 474
- DE-A1- 3 814 723
- DE-A1- 19 525 920
- DE-A1-102004 045 823
- JP-A- S54 128 060

## Beschreibung

Gegenstand der vorliegenden Erfindung ist eine Vorrichtung in Form einer strömungsdynamischen Reaktoranlage zur Aufnahme eines fluiden Mediums. Der Gegenstand des vorliegenden Verfahrens ist die energieoptimierte Erzeugung und strömungsdynamische Behandlung von mindestens einem geführten Fluidwirbel in einer Reaktionskammer.

Dabei wird der Fluidwirbel erzeugt, indem ein fluides Medium in einer Reaktionskammer in Rotation versetzt und durch Richtungsänderung mittels Umlenkung in ein Auslaufrohr, welches einen Venturi-Effekt erzeugen kann, aufgenommen wird. Dabei bildet der geführte Volumenstrom des fluiden Mediums spätestens im Austritt aus der Reaktoranlage einen Fluidwirbel.

Herkömmliche Vorrichtungen und Reaktionsbehälter sowie Verfahren zur strömungsdynamischen Behandlung von fluiden Medien sind beispielsweise aus den Schriften AT 272 278, DE 195 25 920 A1, DE 101 14 936 , JPS 54128060A, DE 102004045823 A1, EP 0392545 A1, DE 3814723 A1 und EP 1 294 474 B2 bekannt.

In AT 272 278 bzw. DE 101 14 936 A1 werden fluide Medien einer Reaktionskammer zugeführt und durch deren geometrische Form in Rotation versetzt, wobei sich die Geschwindigkeit des rotierenden fluiden Mediums aufgrund der geometrischen Form der Reaktionskammer zunächst verringert und in Folge zum Sohlebereich am unteren Ende der Reaktionskammer wieder erhöht. Das sich rotierend zum Sohlebereich der Reaktionskammer bewegende fluide Medium wird am unteren Ende der Reaktionskammer entgegen der bisherigen Strömungsrichtung zu einer Längsachse geleitet nach oben gelenkt, in einem Auslaufrohr aufgefangen und verlässt die Reaktionskammer rotierend unter Bildung eines Hohlwirbels. Im Sohlebereich der Reaktionskammer befinden sich Öffnungen entlang der Längsachse oder unmittelbarer Nähe dazu, so dass über die Hohlwirbelsenke, welche in ihrem Kern einen Unterdruck erzeugt, zusätzliche fluide Medien angesaugt werden können.

In DE 195 25 920 A1 ist eine Ergänzung der Vorrichtung aus AT 272 278 beschrieben, bei welcher das zu reinigende fluide Medium im Wechsel steigend und fallend durch miteinander verbundene Zulaufrohre strömt und im Anschluss nach dem Auslass aus der Reaktionskammer in ein Rohrlabyrinth zur Sedimentation oder zum Abfangen der verdichteten Abprodukte geleitet wird.

Nachteilig an diesen Vorrichtungen ist die komplizierte Ausführung, die Unhandlichkeit für einen gezielten technischen Einsatz, die mangelhafte Flexibilität und Verstellbarkeit der Komponenten bzw. Teile der Reaktoranlage und die daraus folgende schlechte Reproduzierbarkeit der Ergebnisse.

Aus der JPS54128060A ist eine Reaktoranlage für fluide Medien bekannt. Diese Anlage weist einen zentralen Einlauf und radiale Ausläufe auf. Die Anlage dient zur Sedimentation von Materialien.

In EP 1 294 474 B2 ist die Reaktionskammer der Reaktoranlage im Querschnitt herz- oder birnenförmig aufgebaut. Das bis in den Sohlebereich reichende und verstellbare Auslaufrohr entlang der Längsachse der Reaktionskammer ist im mündungsnahen Bereich als Düse zur Realisierung des Venturi-Effekts ausgebildet.

Das fluide Medium wird über mindestens eine der Mantelfläche der Reaktionskammer tangential angeordnete Zuführungsöffnung in die Reaktionskammer zugegeben und bewegt sich beschleunigt rotierend als Fluidwirbel um das Auslaufrohr in Fließrichtung nach unten. Durch die Fluidführung, welche im unteren Gehäusebereich den Volumenstrom, welcher seine absolute Drehrichtung beibehält, zur Längsachse rotierend in sich umlenkt, entsteht ein Bereich gegeneinander reibender rotierender Volumenströme mit jeweils hohen Geschwindigkeiten. Die dabei erreichte relative Geschwindigkeit und die hohe Reibung führen zur mechanischen Zerkleinerung und Zerstörung mitgeführter oder gelöster Stoffe.

Dabei ist die Zuführungsöffnung größer als der kleinste Querschnitt der Düse im mündungsnahen Bereich des Auslaufrohres, wodurch ein Staudruck entsteht. Somit tritt neben der Fluidwirbelbildung, welche Wirbelkern ein Vakuum erzeugt, ein zusätzlicher Vakuumeffekt in translatorischer Richtung aufgrund eines Venturi-Effektes im Auslaufrohr auf. Der Venturi-Effekt wiederum basiert auf der Bernoulli Gleichung p_{ges} = p₀ + *ρ*/2 * c² + *ρgh,* wobei po der statische Druck ist, welcher allseitig in der Strö mung vorhanden ist, *ρ*/2 * c² dem dynamischen Druck, welcher den kinetischen Anteil der Energie mit der Strömungsgeschwindigkeit c entspricht, und *pgh* den geodätischen Druckanteil darstellt. Die Strömungsgeschwindigkeit c wiederum ergibt sich aus dem Produkt der Winkelgeschwindigkeit ω mit dem Radius r, welcher sich im Längsschnitt vom äußersten Punkt der Reaktionskammer zur Außenwand des Auslaufrohres erstreckt (c = ω * r). Die Winkelgeschwindigkeit ω entspricht im Folgenden auch der Rotationsgeschwindigkeit des fluiden Mediums.

Zudem ändert sich durch die hohe Zentrifugalkraft und durch die Reibung die Struktur des fluiden Mediums dahingehend, dass bei flüssigen fluiden Medien eine Änderung der Oberflächenspannung und der Viskosität eintritt. In diesem Zustand tritt das fluide Medium in die Eintrittsöffnung des Auslaufrohres rotierend ein. Dadurch bildet sich ein Wirbelfuß mit einem Wirbelkern hoher Geschwindigkeit, welcher aufgrund der strömungsdynamischen Gesetze ein Vakuum in dessen Mitte erzeugt. Die im unteren mündungsnahen Bereich des Auslaufrohres vorkommende Düse zur Realisierung des Venturi-Effekts führt dazu, dass dieser Vakuumbereich bei entsprechender Strömungsgeschwindigkeit durch die Erzeugung eines zusätzlichen Vakuums überlagert und somit verstärkt wird. Der dabei entstehende Unterdruck kann nach der Bernoulli Gleichung absolut < 10 mbar betragen. Durch Druck und Unterdruck sowie die damit verbundene Wirbelbildung werden sehr hohe mechanische Kräfte im fluiden Medium frei. Diese führen zur Änderung der Struktur des fluiden Mediums, hin zu einer geringeren Oberflächenspannung.

Im fluiden Medium mitgeführte organische Bestandteile wie Bakterien und Keime platzen aufgrund des eigenen inneren Zelldruckes (Turgor) im Unterdruckbereich der Düse mechanisch auf. Die organischen Reste werden durch den geänderten Druckbereich, basierend auf der thermischen Zustandsgleichung idealer Gase, *p* · *V* = *m* · *R* · *T,* zu einer chemischen Reaktion geführt. Mitgeführte Belastungen können je nach notwendiger Reaktionsenthalpie im Unterdruckbereich zur Reaktion geführt werden. Dabei kommt es zur Oxidation des fluiden Mediums mit Oxidationsmitteln wie Sauerstoff oder durch angesaugten Sauerstoff aus der Umgebungsluft. Dies geschieht in Abhängigkeit des Energieeintrages im System auch mit anderen oxidierbaren Stoffen - jedoch ist hier nach der thermischen Zustandsgleichung idealer Gase physikalisch eine Grenze gesetzt.

Bei EP 1 294 474 B2 und DE 195 25 920 A1 ist die Reaktionskammer geometrisch so ausgebildet, dass das rotierende fluide Medium eine Beschleunigung durch Verjüngung der Reaktionskammer in Fließrichtung von der Zuführungsöffnung zur Fluidführung und letztlich zum unteren mündungsnahen Bereich des Auslaufrohres erfährt.

Nachteilig an diesen Erfindungen ist der hohe Energiebedarf um das fluide Medium in Rotation zu versetzen, was mit der Form der und Ausgestaltung der Reaktionskammer zusammen hängt. Damit einher geht der geringe wirtschaftliche Nutzen zur Erzeugung des Unterdruckes und die, trotz überraschend guter Wirkungsweise, begrenzte Enthalpie-Einbringung zur Reaktion chemischer Verbindungen oder organischer Belastungen ohne eigenen inneren Zelldruck (Turgor), wie beispielsweise bei Hefen und Pilzen.

Bei dem in EP 1 294 474 B2 beschriebenen Verfahren und der zugehörigen Anlagenausührung werden zur Erzeugung einer Rotation des fluiden Mediums erhebliche Mengen an Strömungsenergie benötigt, da es zu Verlusten durch entstehende unerwünschte Wirbel aufgrund von Fluidreibung in der Reaktionskammer kommt.

Die Verluste an Strömungsenergie können mehr als 20% betragen. Bei beispielsweise verfügbarem eingangsseitigem Pumpendruck von 6 bar stehen der gesamten Reaktoranlage, je nach Anlagenausführung, ausgangsseitig lediglich 2-3 bar zur Verfügung. Betrachtet man zudem, dass mindestens 1 bar benötigt wird, um den Unterdruck in einer Düse zur Realisierung des Venturi-Effekts zu erzeugen und durch Fluidreibung an den Flächen ebenfalls Druck benötigt wird, belaufen sich die Druckverluste durch Fluidreibung in einem Bereich von mindestens 20-30 %. Zudem entstehen durch die unerwünschten Wirbel große Kavitationsbereiche in der Reaktionskammer, welche zur ungewollten Abrasion der Wände der Reaktionskammer, sowie zur Zerstörung von Bereichen der Reaktionskammer bzw. der Düse als mechanisch schwächstes Glied führen können.

Der vorliegenden Erfindung liegt die Aufgabe zugrunde, eine vorteilhafte Vorrichtung sowie ein vorteilhaftes Verfahren für den Betrieb dieser Vorrichtung vorzuschlagen, welche die Verluste an Strömungsenergie in der Reaktionskammer durch eine strömungstechnisch optimierte geometrische und rotationssymmetrische Gestaltung der Reaktionskammer bis zur Eintrittsöffnung des Auslaufrohres reduziert. Weiterhin soll durch die erfindungsgemäße Vorrichtung bei gleichem Energieverbrauch eine größere Beschleunigung des fluiden Mediums in der Reaktionskammer erreicht werden. Zudem soll die Bildung von unerwünschten, durch Fluidreibung erzeugten Wirbeln in der Reaktionskammer reduziert werden.

Durch die sich ändernden Druckverhältnisse in der Reaktionskammer auf Basis von Wirbelbildungen soll damit einhergehend im erfindungsgemäßen Verfahren der durch die erfindungsgemäße Vorrichtung erzielte Abbau und die mechanische Zerstörung und Zerkleinerung von im fluiden Medium gelösten Fremdstoffen aufgrund der vorhandenen Reibungs- und Zentrifugalkräfte effizienter bewirkt werden.

Dadurch sollen fluide Medien schneller, kostengünstiger, platzsparender, umweltfreundlicher und leistungsstärker gereinigt und aufbereitet werden.

Ferner soll es Aufgabe der Erfindung sein, die erfindungsgemäße Vorrichtung und das erfindungsgemäße Verfahren sowie eine Vorrichtung zur Durchführung des Verfahrens zu verwenden.

Diese Aufgabe wird mit den in den Patentansprüchen 1 und 8 aufgeführten Merkmalen gelöst. Vorteilhafte Ausgestaltungen der erfindungsgemäßen Vorrichtung, des erfindungsgemäßen Verfahrens und der erfindungsgemäßen Verwendung sind in den Ansprüchen 2 bis 7 und 9 bis 11 gegeben.

Erfindungsgemäß wird die Aufgabe gelöst durch eine Reaktoranlage zur strömungsdynamischen Behandlung fluider Medien basierend auf mechanischen, physikalischen und chemischen Vorgängen.

Die Aufgabe wird gelöst durch eine strömungsdynamischen Reaktoranlage gemäß Anspruch 1 J Z zur Aufnahme eines fluiden Mediums zur Erzeugung von mindestens einem geführten Fluidwirbel, umfassend ein Gehäuse und ein Auslaufrohr, wobei das Gehäuse mittels der fluidkontaktseitigen Innenwände einen um eine Längsachse rotationssymmetrischen fluidführenden Hohlraum bildet, der im Folgenden Reaktionskammer genannt wird. Dabei ist die Reaktionskammer in Fließrichtung des fluiden Mediums in einen oberen und einen unteren Teil aufgeteilt. Der obere Teil der Reaktionskammer weist dabei eine Deckfläche und eine Grundfläche auf, sowie einen Übergangsbereich von der Deck- zur Grundfläche. Weiterhin weist der obere Teil der Reaktionskammer im Übergangsbereich von der Deck- zur Grundfläche einen maximalen Radius, bezogen auf die Außenwand des Auslaufrohres, auf. Im Übergangsbereich von der Deckfläche zur Grundfläche ist mindestens eine tangential zur Mantelfläche des oberen Teils der Reaktionskammer angeordnete Zuführungsöffnung mit einen sich in Fließrichtung anschließenden Fluideintrittsbereich. Die Deck- bzw. Grundfläche besitzt einen Anstellwinkel zur Längsachse von 80° bis 115°. Der untere Teil der Reaktionskammer erstreckt sich in Fließrichtung in einem Abstand z vom Übergang der Grundfläche bis zu der unteren Begrenzung eines gekrümmten Sohlebereich, wo eine geometrisch aufsteigend geformte Fluidführung das fluide Medium in eine Eintrittsöffnung des Auslaufrohres umlenkt. Weiterhin fällt das Auslaufrohr in seiner Längsachse mit der Längsachse der rotationssymmetrischen Reaktionskammer zusammen. Die Eintrittsöffnung des Auslaufrohres ist in einem Abstand a zur in Fließrichtung unteren Begrenzung des gekrümmten Sohlebereichs angeordnet.

In die Reaktionskammer der Reaktoranlage wird ein fluides Medium eingeleitet. Fluide Medien bzw. Fluide im Sinne der Erfindung sind flüssige und/oder gasförmige Stoffe und/oder Gemische aus flüssigen und/oder gasförmigen Stoffen.

Bevorzugt ist das fluide Medium eine Flüssigkeit. In einer Ausführungsform wird als fluides Medium mindestens eine reine Flüssigkeit der Reaktoranlage zugeführt. In einer weiteren Ausführungsform wird als fluides Medium mehr als eine Flüssigkeit der Reaktoranlage zugeführt. Besonders bevorzugt ist das fluide Medium eine wässrige Flüssigkeit bzw. wässrige Lösung, d.h. Wasser enthaltend.

In einer weiteren Ausführungsform wird ein Gemisch aus mindestens einer Flüssigkeit und mindestens einem Gas der Reaktoranlage zugeführt. In einer weiteren Ausführungsform wird mehr als ein Gemisch aus mindestens einer Flüssigkeit und mindestens einem Gas der Reaktoranlage zugeführt.

In einer alternativen Ausführung der Erfindung wird mindestens und ausschließlich ein gasförmiger Stoff bzw. ein gasförmiges Gemisch als fluides Medium in der Reaktoranlage behandelt. In einer besonderen Ausführungsform wird mindestens ein Gas der Reaktoranlage zugeführt.

In der Reaktionskammer wird das mindestens eine zugeführte fluide Medium bzw. der mindestens eine gebildete geführte Fluidwirbel strömungsdynamisch behandelt. Unter der strömungsdynamischen Behandlung des fluiden Mediums in der erfindungsgemäßen strömungsdynamischen Reaktoranlage wird verstanden, dass das fluide Medium als Volumenstrom über mindestens eine Zuführungsöffnung und einen sich in Fließrichtung anschließenden Fluideintrittsbereich in die Reaktionskammer geführt wird. Die Fließrichtung bezieht sich dabei stets auf die des fluiden Mediums.
Durch die Geometrie und Gestaltung der Reaktionskammer wird mindestens ein geführter Fluidwirbel gebildet, einhergehend mit einer Wirbelumstülpung des mindestens einen Fluidwirbels und dem Aufplatzen von im fluiden Medium gelösten organischer Bestandteile mit innerem Zelldruck (Turgor). Der mindestens eine erzeugte geführte Fluidwirbel wird somit in der Reaktoranlage strömungsdynamisch behandelt und dabei aufbereitet, gereinigt und desinfiziert.

Die strömungsdynamische Behandlung des mindestens einen geführten Fluidwirbels wird durch die erfindungsgemäße Reaktoranlage und das erfindungsgemäße Verfahren zum Betreiben dieser Reaktoranlage realisiert. Durch die strömungsdynamische Behandlung des mindestens einen erzeugten Fluidwirbels findet bevorzugt die Umsetzung und/oder mechanische und physikalische Zerstörung und/oder Radikalisierung von im fluiden Medium befindlichen chemischen Stoffen oder Mikroorganismen statt.

Durch die Geometrie und Ausgestaltung der erfindungsgemäßen Reaktoranlage, besonders der Reaktionskammer, ganz besonders des oberen Teils der Reaktionskammer, muss vorteilhaft weniger Energie aufgewendet werden bzw. wird Strömungsenergie eingespart und weniger Druck wird benötigt, um das fluide Medium zu beschleunigen. Andererseits werden bei gleichem Energieverbrauch höhere Drehzahlen des fluiden Mediums und somit eine größere Beschleunigung und Effizienz der Reaktoranlage gewährleistet. Daraus begründet sich das verbesserte erfindungsgemäße Verfahren bei der Zerstörung und Zerkleinerung von bspw. Keimen, da in der erfindungsgemäßen Reaktoranlage eine größere Rotationsgeschwindigkeit des Fluidwirbels hervorgerufen wird.

Die Reaktoranlage umfasst mehrere Komponenten bzw. Teile, wie ein Gehäuse und ein Auslaufrohr, auf welche im Folgenden näher eingegangen wird.

Das Gehäuse besteht aus einem stabilen Material und Körper, welcher im Inneren hohl ist. Das Gehäuse bildet mittels der fluidkontaktseitigen Innenwände einen um eine Drehachse rotationssymmetrischen Hohlraum, welcher im Folgenden Reaktionskammer genannt wird. Die Reaktionskammer ist damit rotationssymmetrisch zur Drehachse. Die Drehachse der Reaktionskammer wird im Folgenden als Längsachse bezeichnet.

Alle Angaben, welche im Folgenden über die Komponenten bzw. Teile der Reaktoranlage aufgeführt werden, beziehen sich immer auf eine Hälfte der Reaktoranlage im Längsschnitt. Der Aufbau der zweiten Hälfte der Reaktoranlage auf der anderen Seite der Längsachse ist jedoch derselbe, da die Reaktoranlage im Längsschnitt spiegelsymmetrisch aufgebaut ist.

Die Außenwände des Gehäuses können eine beliebige geometrische Form annehmen. Bevorzugt ist das Gehäuse rotationssymmetrisch ausgeführt.

Im Längsschnitt der Reaktoranlage liegt horizontal (also senkrecht zur Längsachse) eine fiktive Mittelebene. In einer Ausführungsform verläuft die Mittelebene durch den oberen Teil des Gehäuses und der Reaktionskammer. In einer weiteren Ausführungsform verläuft die Mittelebene durch den oberen Teil des Gehäuses und der Reaktionskammer. In einer bevorzugten Ausführungsform verläuft die Mittelebene durch die Mittelpunkte des Fluideintrittsbereichs, welcher sich in Fließrichtung an die mindestens eine Zuführungsöffnung anschließt.

Das Gehäuse ist in Einbaulage bezüglich dieser Mittelebene unterteilt in einen oberen Teil und einen unteren Teil. Der obere Teil des Gehäuses liegt in Einbaulage oberhalb der Mittelebene und der untere Teil des Gehäuses schließt sich unterhalb der Mittelebene in Fließrichtung des fluiden Mediums an.

Unter der Fließrichtung des fluiden Mediums wird die Flussrichtung des in die Reaktoranlage geführten fluiden Mediums verstanden. Das fluide Medium fließt in Einbaulage nach unten und wird an der Fluidführung an der unteren Begrenzung des Sohlebereichs des unteren Teils der Reaktionskammer in Einbaulage nach oben (entgegengesetzt seiner ursprünglichen Richtung) in die Eintrittsöffnung des Auslaufrohres umgeleitet. In einer Ausführungsform verlässt das fluide Medium die Reaktoranlage durch die Austrittsöffnung des Auslaufrohres an einer in Einbaulage höheren Stelle als es in die Reaktoranlage durch die mindestens eine Zuführungsöffnung eingetreten ist.

Das Gehäuse umfasst mindestens zwei Öffnungen. Die mindestens zwei Öffnungen umfassen eine Öffnung für ein Zulaufrohr für den Medienzufluss sowie eine im oberen Teil des Gehäuses entlang der Längsachse zentral angeordneten Öffnung für ein Auslaufrohr für den Medienaustritt. In einer weiteren besonders bevorzugten Ausführungsform weist das Gehäuse darüber hinaus auch mehr als eine Öffnung für mehrere Zulaufrohre und Medienzuflüsse auf, beispielsweise zwei, drei, vier oder mehr Öffnungen.

In einer weiteren bevorzugten Ausführungsform umfasst das Gehäuse eine weitere Öffnung, wobei diese Öffnung eine entlang der Längsachse zentral angeordnete Öffnung in Einbaulage im untersten Teil des Gehäuses für das Einbringen einer Fluidführung darstellt.

Vorteilhaft weist das Gehäuse mindestens eine Öffnung für das Auslaufrohr, mindestens eine Öffnung für das Einbringen einer Fluidführung sowie mindestens eine Öffnung für ein Zulaufrohr auf.

Dadurch kann mehr als ein fluides Medium in den oberen Teil der Reaktionskammer geleitet werden und es wird weniger Kraft für die Einleitung der Volumenströme in die Reaktionskammer aufgewendet. Die Volumenströme können dabei aus den Rohrleitungen eines Hauptzuflusses oder verschiedenen Zuleitungen stammen. Die Volumenströme können weiterhin aus demselben fluiden Medium oder unterschiedlichen fluiden Medien bestehen.
Die Reaktionskammer kann verschiedene Geometrien annehmen. In einer ganz bevorzugten Ausführungsform ist die Reaktionskammer rotationssymmetrisch geformt.

Die um eine Längsachse rotationssymmetrische Reaktionskammer wird durch die Innenwände des Gehäuses geformt. In einer Ausführungsform sind die Innenwände des Gehäuses in Kontakt mit dem fluiden Medium. Die fluidkontaktseitigen Innenwände des Gehäuses werden im Folgenden als Wände der fluidführenden Reaktionskammer bezeichnet. Die Reaktionskammer ist in Fließrichtung des fluiden Mediums in einen oberen und einen unteren Teil aufgeteilt.

Die Reaktionskammer nimmt das durch die mindestens eine Zuführungsöffnung einströmende fluide Medium auf. Das fluide Medium wird als Strömung, im Folgenden auch Volumenstrom genannt, durch die mindestens eine Zuführungsöffnung in einen Fluideintrittsbereich in den oberen Teil der rotationssymmetrischen fluidführenden Reaktionskammer geleitet und bildet im weiteren Verlauf einen Fluidwirbel.
Die Wahl der Strömungsgeschwindigkeit ist abhängig von den jeweiligen Eigenschaften des fluiden Mediums und kann aus der Stärke der Nebenvalenzbindung bzw. der Festigkeit der Moleküle ermittelt werden. Vorteilhaft wird eine hohe Geschwindigkeit zum Einleiten des fluiden Mediums in den oberen Teil der Reaktionskammer gewählt.

Da die Reaktionskammer durch die fluidkontaktseitigen Innenwände des Gehäuses gebildet wird, weist sie analog zum Gehäuse auch dessen Öffnungen auf. Daher umfasst die Reaktionskammer mindestens zwei Öffnungen (eine Öffnung für das Zulaufrohr, welches im Schnitt mit der Mantelfläche des oberen Teils der Reaktionskammer eine tangential angeordnete Zuführungsöffnung für den Medienzufluss bildet und eine Öffnung im oberen Teil der Reaktionskammer entlang der Längsachse zentral angeordnete Öffnung für das Auslaufrohr zum Medienaustritt). In einer weiteren besonders bevorzugten Ausführungsform weist die Reaktionskammer darüber hinaus auch mehr als eine Zuführungsöffnung auf, beispielsweise zwei, drei, vier oder mehr Zuführungsöffnungen.

Die Reaktionskammer und die Öffnungen für Medienzufluss und Medienaustritt sind derart gestaltet und zueinander angeordnet, dass in dem zu behandelnden fluiden Medium beim Durchströmen der Reaktionskammer von der mindestens einen Zuführungsöffnung zur Austrittsöffnung möglichst große, durch Reibung der einzelnen Strömungsschichten untereinander und mit den Wänden der Reaktionskammer erzeugte Schubspannungen erzeugt werden.
In einer weiteren bevorzugten Ausführungsform umfasst die Reaktionskammer eine weitere Öffnung, wobei diese Öffnung eine entlang der Längsachse zentral angeordnete Öffnung an der unteren Begrenzung des Sohlebereichs der Reaktionskammer für das Einbringen einer Fluidführung darstellt.

Ganz bevorzugt weist die Reaktionskammer mindestens eine Öffnung für das Auslaufrohr, mindestens eine Öffnung für das Einbringen einer Fluidführung sowie mindestens eine Zuführungsöffnung auf.

Bevorzugt weist die Reaktionskammer zwei Zuführungsöffnungen auf. Dadurch werden bevorzugt zwei oder mehr als zwei Volumenströme in die Reaktionskammer geleitet. Dabei ist die Geschwindigkeit der Volumenströme so zu wählen, dass sich strömungstechnisch eine turbulente Grenzschicht ausbilden kann und die Volumenströme eine hohe Geschwindigkeitsdifferenz aufweisen. Vorzugsweise wird eine Kombination aus Translationsbewegung und gleichzeitiger Rotationsbewegung so gewählt, dass sich die Volumenströme berühren.

In einer Ausführungsform sind alle Wände der Reaktionskammer in Kontakt mit dem durch die mindestens eine Zuführungsöffnung eingeleiteten fluiden Medium. In einer alternativen Ausgestaltung ist nur ein Teil der Wände der Reaktionskammer in Kontakt mit dem durch die mindestens eine Zuführungsöffnung eingeleiteten fluiden Medium.

Die Reaktionskammer ist in Einbaulage entlang der Längsachse in Fließrichtung aufgeteilt in einen oberen Teil und einen unteren Teil, welche jeweils rotationssymmetrisch sind. Erfindungsgemäß wird unter dem oberen Teil der Reaktionskammer jener Teil verstanden, in welchem das fluide Medium durch die mindestens eine Zuführungsöffnung eingeleitet wird. Dabei erstreckt sich der obere Teil der Reaktionskammer, entlang einer Mittelebene betrachtet, von der mindestens einen Zuführungsöffnung für den Medienzufluss bis zu einer Außenwand des Auslaufrohres.

In einer Ausführungsform weist der obere Teil der Reaktionskammer eine Deckfläche und eine Grundfläche auf, welche jeweils durch die Wände der Reaktionskammer gebildet werden.

Die Deckfläche umfasst dabei jene Fläche, die von der Wand der oberen Reaktionskammer von dem in Einbaulage oberen Bereich der mindestens einen Zuführungsöffnung und dem sich anschließenden Fluideintrittsbereich bis zum Abschluss mit der Außenwand des Auslaufrohres reicht. Die Grundfläche wird von der Wand der oberen Reaktionskammer gebildet und umfasst die Fläche, welche sich von dem in Einbaulage unteren Bereich der mindestens einen Zuführungsöffnung und dem sich anschließenden Fluideintrittsbereich zum unteren Teil der Reaktionskammer erstreckt.

Bevorzugt ist als Radius *r*₁ jener Abstand definiert, der von der Grundfläche des oberen Teils der Reaktionskammer zur Außenwand des Auslaufrohres entlang einer Ebene parallel zur Mittelebene verläuft. Weiterhin werden die bei EP 1 294 474 B2 nachteiligen Druckverluste umgangen, indem in der vorliegenden Erfindung der Radius *r*₁ in Fließrichtung konstant bleibt bzw. sich kontinuierlich verringert. In einer bevorzugten Ausführungsform ist *r*₁ deutlich größer als der Abstand b zwischen Grund- und Deckfläche.

Weiterhin weist der obere Teil der Reaktionskammer einen Übergangsbereich von der Deckfläche zur Grundfläche auf. Bevorzugt stellt der Übergangsbereich von der Deck- zur Grundfläche im Längsschnitt der Reaktoranlage einen Kreissektor oder Ellipsensektor dar. In einer weiteren Ausführungsform kann der Übergangsbereich von der Deck- zur Grundfläche verschiedene geometrische Formen aufweisen. Der Übergangsbereich von der Deck- zur Grundfläche stellt weiterhin im Längsschnitt der Reaktoranlage den am weitest entferntesten Punkt der Reaktionskammer zur Außenwand des Auslaufrohres dar.

Der Abstand vom Übergangsbereich von der Deckfläche zur Grundfläche im oberen Teil der Reaktionskammer zur Außenwand des Auslaufrohres entlang der Mittelebene stellt den maximalen Radius der Reaktionskammer dar und wird im Folgenden als maximaler Radius *r*ₘₐₓ bezeichnet. Dabei verläuft *r*ₘₐₓ in einer Ausführungsform entlang der Mittelebene, d.h. vom Übergangsbereich zwischen Deck- und Grundfläche des oberen Teils der Reaktionskammer durch den Mittelpunkt des Fluideintrittsbereiches zur Außenwand des Auslaufrohres. Entsprechend des Drehimpulssatzes *L* = *mcr* mit dem Massestrom *ṁ* kann über die erreichte Winkel- bzw. Rotationsgeschwindigkeit nach der Bernoulli Gleichung und den Wirbelgesetzen der Dampfdruck des fluiden Mediums im Bereich von *r*ₘₐₓ nicht erreicht werden. Dabei gilt für den Massestrom *ṁ* = *V̇ · ρ,* wobei *V̇* den Volumenstrom und *p* die Dichte des fluiden Mediums darstellt. Für einen konstanten Massestrom *ṁ* sowie einen konstanten Drehimpuls *L* wiederum erhöhen sich die Drehzahlen und somit die Winkelgeschwindigkeit *ω* des fluiden Mediums bei einer Reduzierung von *r*ₘₐₓ deutlich.

Der obere Teil der Reaktionskammer weist im Übergangsbereich von der Deckfläche zur Grundfläche mindestens eine tangential zum Querschnitt der Mantelfläche des oberen Teils der Reaktionskammer eintretende Zuführungsöffnung auf, durch welche das fluide Medium in die Reaktionskammer geleitet wird.
In einer bevorzugten Ausgestaltung ist die mindestens eine Zuführungsöffnung somit am maximal ausgeprägten Abstand *r*ₘₐₓ des oberen Teils der Reaktionskammer zwischen Übergangsbereich zwischen Deck- und Grundfläche und Außenwand des Auslaufrohres entlang der Mittelebene angeordnet, wodurch vorteilhaft ein längerer Beschleunigungsweg für das fluide Medium im oberen Teil der Reaktionskammer bereitgestellt wird.

An die mindestens eine Zuführungsöffnung schließt sich in Fließrichtung des fluiden Mediums ein Fluideintrittsbereich im oberen Teil der Reaktionskammer an, welcher im Längsschnitt zur Reaktoranlage eine kreisförmige Fläche mit einen Durchmesser *d*_{z} aufweist.

In einer ersten erfindungsgemäßen Ausführungsform weisen die Deckfläche und die Grundfläche des oberen Teils der Reaktionskammer in Fließrichtung ab dem Übergangsbereich von der Deck- zur Grundfläche bis zum Übergang der Grundfläche in den unteren Teil der Reaktionskammer einen weitestgehend konstanten Abstand b zueinander auf. Ist der Abstand b konstant, entspricht der konstante Abstand b dabei gleichzeitig dem maximalen Abstand *b*ₘₐₓ zwischen Deck- und Grundfläche (im Längsschnitt der Reaktoranlage gesehen).

Für eine vorteilhafte Beschleunigung des fluiden Mediums, entspricht der konstante Abstand der Deck- zu der Grundfläche dem einfachen Durchmesser *d*_{z} des Fluideintrittsbereiches (*b* = *d*_{z}), wodurch der obere Teil der Reaktionskammer einen relativ schlanken und flachen Bereich für das einströmende fluide Medium darstellt. In diesem Fall erscheint der obere Teil der Reaktionskammer im Längsschnitt der Reaktoranlage wie eine Scheibe bzw. tellerförmig.

In einer zweiten erfindungsgemäßen Ausführungsform weisen die Deck- und die Grundfläche in Fließrichtung des fluiden Mediums ab dem Übergangsbereich von der Deck- zur Grundfläche bis zu einem Übergang der Grundfläche in den unteren Teil der Reaktionskammer einen sich verringernden Abstand b zueinander auf. Der Abstand b verringert sich in Fließrichtung des fluiden Mediums kontinuierlich in Richtung Auslaufrohr. Dabei ist der Abstand der Deck- zur Grundfläche bei der mindestens einen Zuführungsöffnung und dem sich in Fließrichtung anschließenden Fluideintrittsbereich maximal (*b*ₘₐₓ) und entspricht dem einfachen Durchrresser *d*_{z} des Fluideintrittsbereiches (*b* = *d*_{z}). Bei dem sich verringernden Abstand b verläuft eine fiktive Zwischenebene durch den Mittelpunkt des Fluideintrittsbereiches parallel zur der Deckfläche des oberen Teils der Reaktionskammer.

Durch den sich verringernden Abstand b wird vorteilhaft, basierend auf dem Drehimpulssatz, eine größere Beschleunigung des eingeführten fluiden Mediums erreicht. Weiterhin führt diese zusätzliche Verjüngung des oberen Teils der Reaktionskammer zu einer erhöhten Viskosität des fluiden Mediums, *E* = *V̇ l b.* Dabei ist *V̇* der Volumenstrom und b der Abstand zwischen Deck- und Grundfläche.

Die Kontinuitätsgleichung für den Volumenstrom besagt, dass ein Volumenstrom in einer Leitung immer konstant ist. Dies ändert sich auch nicht, wenn sich der Querschnitt der Leitung verändert. Dies wird auch als Venturi-Effekt bezeichnet und bildet die Basis für das Gesetz von Bernoulli. Basierend auf der Kontinuitätsgleichung *V̇* = *c · A* (mit dem Volumenstrom *V̇*, der mittleren Strömungsgeschwindigkeit c und der Querschnittsfläche *A* an der betrachteten Stelle) erhöht sich die mittlere Strömungsgeschwindigkeit mit abnehmender Querschnittsfläche, wodurch es zu einer Erhöhung des Drehimpulses kommt.

Nimmt man den Fluideintrittsbereich als eine Strömungsebene *b*₀ an, so ist die darunter oder nachfolgende Strömungsebene *b*₁ im Längsschnitt im Radius *r* vom äußersten Punkt der Reaktionskammer zur Außenwand des Auslaufrohres dieser nachfolgenden Strömungsebene *b*₁ so zu wählen, dass die sich ergebende Winkelgeschwindigkeit *ω*₁ mindestens 1,5 mal höher liegt als die Winkelgeschwindigkeit *ω*₀ am Fluideintrittsbereich.

Bevorzugt weist der obere Teil der Reaktionskammer in der Draufsicht eine Kreisscheibe oder eine Tellerform auf.

Erfindungsgemäß besitzen die Deck- bzw. Grundfläche einen Anstellwinkel *α* von 80° bis 115°, bevorzugt von 90° bis 110°, ganz besonders bevorzugt von 90°.

Der Anstellwinkel bezieht sich dabei auf jenen Winkel, welcher sich, im Längsschnitt in Einbaulage gesehen, zur Längsachse der Reaktionskammer einstellt.

Der Anstellwinkel bei *α* = 90° stellt sich von der Mittelebene zur Längsachse ein, wobei dabei die Mittelebene durch die Mittelpunkte des Fluideintrittsbereichs verläuft. Dies gilt sowohl für einen gleich bleibenden Abstand *b* zwischen Deck- und Grundfläche (die Mittelebene verläuft dann parallel zu beiden), als auch für einen sich verringernden Abstand *b* zwischen Deck- und Grundfläche. Der Anstellwinkel bei *α* = 90° bezieht sich immer auf den sich in Einbaulage einstellenden Winkel unterhalb der Mittelebene, d.h. von der Mittelebene zur Längsachse der Reaktionskammer. Dazu stellt der Schnitt der Längsachse mit der Mittelebene ein kartesisches Koordinatensystem dar. Der Anstellwinkel *α* = 90° bezieht sich somit immer auf den dritten und/oder vierten Quadranten des kartesischen Koordinatensystems.

Die Anstellwinkel *α* > 90° oder *α* < 90° stellen sich von der fiktiven Zwischenebene zur Längsachse ein, wobei die fiktive Zwischenebene durch die Mittelpunkte des Fluideintrittsbereiches und parallel zur der Deckfläche des oberen Teils der Reaktionskammer verläuft. Dies gilt sowohl für einen gleich bleibenden Abstand *b* zwischen Deck- und Grundfläche (die fiktive Zwischenebene verläuft dann parallel zu beiden), als auch für einen sich verringernden Abstand *b* zwischen Deck- und Grundfläche. Die Anstellwinkel *α* > 90° oder *α* < 90° beziehen sich immer auf den sich in Einbaulage einstellenden Winkel unterhalb der fiktiven Zwischenebene, d.h. von der fiktiven Mittelebene zu Längsachse der Reaktionskammer. Bei einem Anstellwinkel *α* < 90° wird etwas mehr Druck als für *α* > 90° benötigt. Bei einem Anstellwinkel *α* > 90° weist das eingeführte fluide Medium eine in Fließrichtung nach unten fallende Einströmrichtung in die Reaktionskammer auf.

Bei einem Anstellwinkel von *α* = 90° bleibt das eingeleitete fluide Medium auf einer Ebene, und nimmt erst beim Übergang in den unteren Teil der Reaktionskammer eine fallende Bewegung in Fließrichtung ein. Der Abstand *b* entspricht bei einem Anstellwinkel von *α* = 90° der Höhe des oberen Teils der Reaktionskammer in Einbaulage.

In einer Ausführungsform weist der Anstellwinkel *α* für beide Hälften der Reaktoranlage (d.h. links und rechts von der Längsachse) im Längsschnitt dieselben Werte auf. Bevorzugt ist Aufbau der zweiten Hälfte der Reaktoranlage auf der anderen Seite der Längsachse derselbe, da die Reaktoranlage im Längsschnitt spiegelsymmetrisch aufgebaut ist. In einer alternativen Ausführungsform sind die Werte des Anstellwinkel *α* auf beiden Hälften der Reaktoranlage im Längsschnitt unterschiedlich.

Durch die erfindungsgemäße rotationssymmetrische Teller- bzw. Tassenform des oberen Teils der Reaktionskammer im Längsschnitt der Reaktoranlage werden die Eigenschaften und Leistungen zur strömungsdynamischen Behandlung von fluiden Medien, die in der Reaktionskammer eine hohe Reibung des fluiden Mediums bis zur Fluidführung aufweisen, stark verbessert. Die Nachteile der Fluidwirbelbildung in der herzförmigen Reaktoranlage, wie bspw. in EP 1 294 474 B2 offenbart, werden verringert oder völlig aufgehoben.

Unter dem unteren Teil der Reaktionskammer wird jener Teil verstanden, der dem oberen Teil der Reaktionskammer in Einbaulage und in Fließrichtung des fluiden Mediums nachfolgt und von den fluidkontaktseitigen Innenwänden des unteren Teils des Gehäuses gebildet wird.

Der untere Teil der Reaktionskammer weist eine Grundfläche auf, welche sich an die Grundfläche des oberen Teils der Reaktionskammer anschließt.

Die Grundfläche des unteren Teils der Reaktionskammer beginnt ab jener Stelle, an welchem der Abstand b zwischen Deck- und Grundfläche des oberen Teils der Reaktionskammer nicht mehr konstant ist bzw. sich verringert, sondern größer wird. Im Folgenden wird diese Stelle als Übergang der Grundfläche des unteren Teils der Reaktionskammer beschrieben. Der Übergang der Grundfläche des unteren Teils der Reaktionskammer umfasst dabei lediglich die vom oberen Teil der Reaktionskammer kommende Grundfläche und keine Deckfläche mehr, da die Deckfläche bereits im oberen Teil der Reaktionskammer in der Außenwand des Auslaufrohres mündet.

Der Abstand, welcher sich von der Außenwand des Auslaufrohres zum Beginn des Übergangs der Grundfläche des unteren Teils der Reaktionskammer in Fließrichtung erstreckt, entspricht dem Radius *r*₃. Dabei kann *r*₃ niemals den Radius *r*₁ oder den maximalen Radius *r*ₘₐₓ des oberen Teils der Reaktionskammer erreichen oder überschreiten.

In einer bevorzugten Ausführungsform ist *r*₁ mindestens doppelt so groß wie der Durchmesser *d*_{z} des Fluideintrittsbereiches (*r*₁ ≥ ½ *d*_{z}). In einer ganz besonders bevorzugten Ausführungsform ist *r*₁ mindestens größer als die Summe des Durchmessers des Fluideintrittsbereiches *d*_{z} und des Abstandes *r*₃ vom Übergang der Grundfläche des unteren Teils des Reaktionskammer zur Außenwand des Auslaufrohres (*r*₁ ≥ *d_{z}* + *r*₃)*.*

In einer Ausführungsform nimmt der Übergang der Grundfläche des unteren Teils der Reaktionskammer eine beliebige Kontur an. In einer bevorzugten Ausführungsform nimmt der Übergang der Grundfläche des unteren Teils der Reaktionskammer eine Krümmung ein. In einer weiteren Ausführungsform knickt der Übergang der Grundfläche des unteren Teils der Reaktionskammer abrupt von der Grundfläche des oberen Teils der Reaktionskammer zur Längsachse hin ab.

In einer Ausführungsform weist der untere Teil der Reaktionskammer in Fließrichtung des fluiden Mediums einen vom Übergang der Grundfläche des unteren Teils der Reaktionskammer zum Sohlebereich des unteren Teils der Reaktionskammer abnehmenden Abstand zur Außenwand des Auslaufrohres auf.
Bevorzugt ist der abnehmende Abstand kontinuierlich. Vorteilhaft wird das fluide Medium dadurch schneller beschleunigt und es kommt zu weniger Druckverlusten in der Reaktionskammer.

In einer alternativen Ausführungsform weist der untere Teil der Reaktionskammer in Fließrichtung des fluiden Mediums einen vom Übergang der Grundfläche zum Sohlebereich abrupt abnehmenden Abstand zur Außenwand des Auslaufrohres auf.

Der untere Teil der Reaktionskammer erstreckt sich in Fließrichtung des fluiden Mediums vom Übergang der Grundfläche des unteren Teils der Reaktionskammer bis zu einer unteren Begrenzung des Sohlebereichs.

Der Sohlebereich des unteren Teils der Reaktionskammer bildet die in Einbaulage untere Begrenzung des unteren Teils der Reaktionskammer und setzt sich als Wand vom Übergang der Grundfläche des unteren Teils der Reaktionskammer fort.

In einer Ausführungsform beginnt der Sohlebereich in Fließrichtung des fluiden Mediums ab einer Krümmung des Übergangs der Grundfläche des unteren Teils der Reaktionskammer.

In einer Ausführungsform nimmt die Wand des Sohlebereichs des unteren Teils der Reaktionskammer eine beliebige Kontur an. Bevorzugt ist der Sohlebereich gekrümmt. Besonders bevorzugt ist der Sohlebereich konkav gekrümmt. Dabei wird unter einer konkaven Krümmung eine im Längsschnitt nach außen, d.h. eine in Einbaulage nach unten ragende Wölbung verstanden. In einer alternativen Ausführungsform ist der Sohlebereich als Paraboloid ausgestaltet. In einer weiteren alternativen Ausführungsform weist der Sohlebereich eine andere Kontur, bspw. eine eckige Kontur, auf.

Durch den bevorzugten gekrümmten Sohlebereich wird der Verlauf der Wände des unteren Teils der Reaktionskammer umgekehrt und das fluide Medium in seiner Strömungsrichtung umgelenkt. Vorteilhaft wird dabei der Großteil der im fluiden Medium zu behandelnden Bestandteile wie bspw. organische Bestandteile aufgrund der Umlenkung des Fluidwirbels zum Platzen gebracht.

Der gekrümmte Sohlebereich des unteren Teils der Reaktionskammer umfasst in Einbaulage eine untere Begrenzung, welcher entlang des unteren Bereichs des unteren Teils der Reaktionskammer verläuft.

Als Abstand z wird dabei jener Abstand definiert, in welchem sich der untere Teil der Reaktionskammer vom Übergang der Grundfläche des unteren Teils der Reaktionskammer, also ab jener Stelle, an welchem der Abstand b zwischen Deck- und Grundfläche des oberen Teils der Reaktionskammer nicht mehr konstant ist bzw. sich verringert, sondern größer wird, bis zur unteren Begrenzung des gekrümmten Sohlebereiches erstreckt.

In einer Ausführungsform ist der Abstand z variabel. In einer bevorzugten Ausführungsform beträgt der Abstand *z* mindestens die Hälfte des Durchmessers des Fluideintrittsbereiches *d*_{z} (*z* ≥ ½ *d*_{z}), um vorteilhaft Reibung innerhalb des Fluidwirbels zu erzeugen.

In einer Ausführungsform in der unteren Begrenzung des Sohlebereichs des unteren Teils der Reaktionskammer eine geometrisch aufsteigend geformte Fluidführung eingesetzt, deren Längsachse mit der Längsachse der rotationssymmetrischen Reaktionskammer zusammen fällt. Im Falle einer eingesetzten Fluidführung erstreckt sich die Kontur des unteren Teils der Reaktionskammer von der unteren Begrenzung des Sohlebereichs fortsetzend bis zur Fluidführung bzw. deren Ausstülpung.

Die mindestens eine Zuführungsöffnung befindet sich im oberen Teil der Reaktionskammer. Durch die mindestens eine Zuführungsöffnung findet somit der Medienzufluss in den oberen Teil der Reaktionskammer statt.

Bevorzugt weist das Gehäuse mindestens eine Öffnung für ein Zulaufrohr auf, wodurch das fluide Medium im mindestens einen Zulaufrohr durch die mindestens eine tangential zum Querschnitt der Mantelfläche gebildete und angeordnete Zuführungsöffnung in den oberen Teil der Reaktionskammer geleitet wird. Vorteilhaft muss durch nur eine Zuführungsöffnung weniger Energie für den Medienzufluss in die Reaktionskammer aufgewendet werden.

In einer bevorzugten Ausführungsform wird das fluide Medium durch mehr als eine tangential zur Mantelfläche des oberen Teils der Reaktionskammer angeordnete Zuführungsöffnung in den oberen Teil der Reaktionskammer eingeleitet, beispielsweise durch zwei, drei, vier oder mehr Zuführungsöffnungen. In einer weiteren bevorzugten Ausführungsform weist der obere Teil der Reaktionskammer zwei Zuführungsöffnungen auf, welche zum Längsschnitt im oberen Teil der Reaktionskammer gegenüber liegend angeordnet sind.

Das zu behandelnde fluide Medium wird aus einem außerhalb der Reaktoranlage liegenden Zulaufrohr durch die Öffnung im Gehäuse und der sich anschließenden gebildeten mindestens einen Zuführungsöffnung in den oberen Teil der rotationssymmetrischen Reaktionskammer eingeleitet. Das Zulaufrohr für den Medienzufluss entspricht dem Hauptzufluss oder von diesem abzweigenden Rohrleitungen.

Das Zulaufrohr führt durch die Öffnung im Gehäuse und schneidet die Mantelfläche des oberen Teils der Reaktionskammer im Querschnitt tangential, wodurch ein schräg abgeschnittener Kreiszylinder und somit die mindestens eine Zuführungsöffnung gebildet wird. Das zu behandelnde fluide Medium tritt somit tangential zum Querschnitt der Mantelfläche des oberen Teils der Reaktionskammer durch die mindestens eine Zuführungsöffnung in den oberen Teil der Reaktionskammer ein.

In einer Ausführungsform stellt das Zulaufrohr eine Rohrzuleitung und somit einen länglichen Hohlkörper, bevorzugt ein Rundrohr mit einer Kreisfläche im Querschnitt, dar. Die mindestens eine Zuführungsöffnung weist durch den Schnitt des Zulaufrohres mit der Mantelfläche des oberen Teils der Reaktionskammer eine kreisförmige oder elliptische Fläche auf.

An die mindestens eine Zuführungsöffnung schließt sich in Fließrichtung im oberen Teil der Reaktionskammer ein Fluideintrittsbereich an, welcher das durch die mindestens eine Zuführungsöffnung in die Reaktionskammer einströmende fluide Medium aufnimmt und weiterleitet. Der Fluideintrittsbereich weist einen Durchmesser *d*_{z} auf. In einer bevorzugten Ausführungsform sind die mindestens eine Zuführungsöffnung und der sich in Fließrichtung anschließende Fluideintrittsbereich im Übergangsbereich von der Deck- zur Grundfläche angeordnet. In einer ganz besonders bevorzugten Ausführungsform ist der Mittelpunkt des Fluideintrittsbereiches entlang der Mittelebene angeordnet.

In einer Ausführungsform wird genau ein fluides Medium durch eine Zuführungsöffnung in den sich anschließenden Fluideintrittsbereich des oberen Teils der Reaktionskammer geleitet. In einer alternativen Ausführungsform wird mehr als ein fluides Medium in die Reaktionskammer geleitet, bevorzugt jeweils durch eine Zuführungsöffnung. Alternativ wird mehr als ein fluides Medium durch dieselbe Zuführungsöffnung in die Reaktionskammer geleitet. Die fluiden Medien können dabei identisch oder unterschiedlich sein. Die fluiden Medien können dabei aus vom Hauptzufluss oder von diesem abzweigenden Rohrleitungen oder anderen Zulaufrohren stammen.

Das Auslaufrohr ist ein als durchgehender Hohlzylinder ausgebildetes Rohr und wird in einer im oberen Teil des Gehäuses im Längsschnitt der Reaktoranlage entlang der Längsachse zentral angeordneten Öffnung dichtend eingeführt.

In einer besonderen Ausführungsform besteht das Auslaufrohr aus mehreren zylinderförmigen hohlen Teilen. Im Sinne der Erfindung wird das Auslaufrohr in einen oberen Teil und in einen unteren Teil unterteilt.

In einer Ausführungsform ist das Auslaufrohr gegenüber der zentral angeordneten Öffnung im Gehäuse und somit gegenüber der Reaktionskammer entlang der Längsachse verschiebbar und verstellbar und passt sich somit vorteilhaft an die Eigenschaften und Behandlung des fluiden Mediums an. Die Verstellung des Auslaufrohres erfolgt über eine mechanische Verstelleinheit. Das Auslaufrohr ist in einem fest mit dem Gehäuse verbundenen oder relativ dazu fixierten Axiallager so aufgenommen, sodass eine Verstellung entlang der Längsachse auch während des Betriebes möglich ist, ohne dass sich die Position der Schnittstelle zwischen dem Gehäuse und der Rohrleitung des Hauptzuflusses oder anderer Zuleitungen ändert. Dadurch ist eine Anpassung der Betriebsparameter jederzeit bedarfsgerecht und ohne großen Aufwand möglich.

Das Auslaufrohr fällt in seiner Längsachse mit der Längsachse der rotationssymmetrischen Reaktionskammer zusammen. Das Auslaufrohr weist, gemessen von der Längsachse zur Außenwand des Auslaufrohres, einen Radius *r*₂ auf. In einer Ausführungsform ist der Abstand *r*₂ an allen Stellen des Auslaufrohres konstant. In einer alternativen Ausführungsform ist der Abstand *r*₂ an verschiedenen Stellen des Auslaufrohres nicht konstant. Dies liegt an einem sich ändernden Abstand von der Außenwand zur Innenwand des Auslaufrohres, welcher als Wanddicke d bezeichnet wird.

In einer Ausführungsform sind die Innenwände des Auslaufrohres in Kontakt mit dem fluiden Medium und somit fluidführend.

Der in Einbaulage obere Teil des Auslaufrohres befindet sich im oberen Teil der Reaktionskammer bzw. außerhalb des Gehäuses. Das Auslaufrohr weist im oberen Teil einen oberen mündungsnahen Bereich auf, welcher aus dem Gehäuse ragt und als Schaurohr ausgeführt sein kann. Das Ende des oberen mündungsnahen Bereichs ist als Austrittsöffnung für das fluide Medium ausgebildet und befindet sich außerhalb des Gehäuses. Hierfindet der Austritt des fluiden Mediums (Medienaustritt) aus der Reaktoranlage statt.

Der Gesamtquerschnitt der Austrittsöffnung setzt sich zusammen aus dem freien Querschnitt und der Wanddicke des Auslaufrohres: *d*_{ges} = 2 · *r*₂ = 2 · (*d* + *d*_{frei}). Der freie Querschnitt *d*_{frei} der Austrittsöffnung, durch welchen das umgelenkte fluide Medium das Auslaufrohr verlässt, stellt dabei den Abstand zwischen den zwei sich gegenüberliegenden fluidkontaktseitigen Innenwänden des Auslaufrohres am Ende des oberen mündungsnahen Bereichs dar und berechnet sich aus der Differenz des Gesamtquerschnitts der Austrittsöffnung mit den Wanddicken: *d*_{frei} = *d*_{ges}- (2 · *d*) = (2 · *r*₂) - (2 · *d*)*.*

Der in Einbaulage untere Teil des Auslaufrohres befindet sich größtenteils im unteren Teil der Reaktionskammer bzw. im Sohlebereich des unteren Teils der Reaktionskammer. Das Auslaufrohr weist im unteren Teil einen unteren mündungsnahen Bereich auf, welcher sich in Fließrichtung des fluiden Mediums an die Eintrittsöffnung des Auslaufrohres anschließt. Der mündungsnahe Bereich des unteren Teils des Auslaufrohres reicht entlang seiner Längsachse bis fast an die untere Begrenzung des Sohlebereichs des unteren Teils der Reaktionskammer. Das Ende des unteren mündungsnahen Bereichs ist als eine senkrecht zur Längsachse eben angeordnete Eintrittsöffnung für das am Sohlebereich umgelenkte fluide Medium ausgestaltet.

Der Gesamtquerschnitt der Eintrittsöffnung setzt sich zusammen aus dem freien Querschnitt und der Wanddicke des Auslaufrohres: *d*_{ges} = 2 · *r*₂ = 2 ·(*d* + *d*_{frei}). Der freie Querschnitt *d*_{frei} der Eintrittsöffnung, durch welche das umgelenkte fluide Medium in das Auslaufrohr gelangt, stellt dabei den Abstand zwischen den zwei sich gegenüberliegenden fluidkontaktseitigen Innenwänden des Auslaufrohres am Ende des unteren mündungsnahen Bereichs dar und berechnet sich aus der Differenz des Gesamtquerschnitts der Eintrittsöffnung mit den Wanddicken: *d*_{frei} = *d*_{ges} - (2 · *d*) = (2 · *r*₂) - (2 · *d*). Bevorzugt weisen die Eintrittsöffnung und die Austrittsöffnung den gleichen Wert des freien Querschnitts *d*_{frei} auf. Weiterhin bevorzugt verkleinert sich der freie Querschnitt *d*_{frei} nur im Bereich der Düse zur Realisierung des Venturi-Effekts.

Die Eintrittsöffnung des Auslaufrohres ist erfindungsgemäß in einem Abstand a zur in Fließrichtung unteren Begrenzung des gekrümmten Sohlebereichs des unteren Teils der Reaktionskammer angeordnet. In einer Ausführungsform ist die Eintrittsöffnung in einem variablen Abstand a zur unteren Begrenzung des gekrümmten Sohlebereichs angeordnet. In einer weiteren Ausführungsform ist die Eintrittsöffnung in einem geringen Abstand a zur unteren Begrenzung des gekrümmten Sohlebereichs angeordnet. Bevorzugt ist der Abstand a zwischen der Eintrittsöffnung des Auslaufrohres und der in Fließrichtung unteren Begrenzung des gekrümmten Sohlebereichs kleiner als der Durchmesser *d*_{z} des Fluideintrittsbereiches (*a* < *d*_{z}).

In einer Ausführungsform ist der Abstand *a* zwischen Eintrittsöffnung und unterster Begrenzung des in Fließrichtung unteren Teils der Reaktionskammer gleich oder kleiner als der Gesamtquerschnitt *d*_{ges} der Eintrittsöffnung.

An die Eintrittsöffnung des Auslaufrohres schließt sich in Fließrichtung des fluiden Mediums der untere mündungsnahe Bereich des Auslaufrohres an. In einer Ausführungsform ist das Auslaufrohr in diesem Bereich im Inneren als Hohlrohr gestaltet, mit einem gleichbleibenden Abstand zwischen den zwei sich gegenüberliegenden fluidkontaktseitigen Innenwänden. Bevorzugt entspricht der gleichbleibende Abstand dem freien Querschnitt *d*_{frei}. Besonders bevorzugt weisen die Eintrittsöffnung, die Austrittsöffnung und der Bereich dazwischen (d.h. zwischen unterem und oberem mündungsnahen Bereich) den gleichen Wert des freien Querschnitts *d*_{frei} auf.
In einer bevorzugten Ausführungsform ist der mündungsnahe Bereich des Auslaufrohres als Düse zur Realisierung des Venturi-Effektes ausgebildet, im Folgenden auch nur Düse genannt. Zur Realisierung des Venturi-Effektes weisen die fluidkontaktseitigen Innenwände des Auslaufrohres eine engste Stelle auf, welche die Düse formen. Dies ist in der Regel die Stelle mit dem kleinsten freien Querschnitt der fluidkontaktseitigen Innenwände des Auslaufrohres. Dies wiederum führt zu einer Vergrößerung der Wanddicke d des Auslaufrohres.

Wenn der Gesamtquerschnitt *d*_{ges} der Eintrittsöffnung des Auslaufrohres kleiner als der Durchmesser des Fluideintrittsbereiches ist (*d*_{ges} < *d*_{z}), so sinkt, basierend auf der Bernoulli Gleichung, der Druck an der Eintrittsöffnung des Auslaufrohres. Ist der untere mündungsnahe Bereich des Auslaufrohres als Düse mit dem kleinsten freien Querschnitt ausgebildet, dann stellt sich der Druck so ein, dass sich in der Düse zur Realisierung des Venturi-Effektes ein Unterdruck einstellt.

Vorteilhaft ist die Düse zur Realisierung des Venturi-Effektes im Falle von Reinigung, Beschädigungen oder Defekten austauschbar. In einer bevorzugten Ausführungsform ist die Düse als Venturidüse gestaltet. In einer weiteren bevorzugten Ausführungsform ist die Düse als Lavaldüse gestaltet.

Die Fluidführung besteht aus einem (massiven) Körper. In einer alternativen Ausführungsform besteht die Fluidführung aus mehreren Komponenten. Wird im Folgenden von Fluidführung geschrieben, bezieht sich dies immer auf den in Einbaulage oberen Teil der gesamten Fluidführungskomponente, welcher in den unteren Teil der Reaktionskammer eingeführt ist.

In einer Ausführungsform wird die Fluidführung durch eine zentral angeordnete Öffnung im in Einbaulage untersten Teil des Gehäuses dichtend in die untere Begrenzung des Sohlebereichs des unteren Teils der Reaktionskammer eingeführt. In einer alternativen Ausführungsform ist die Fluidführung ein Teil des Gehäuses und somit bereits fest in dessen untersten Teil integriert. In einer Ausführungsform fällt die Längsachse der Fluidführung mit der Längsachse der rotationssymmetrischen Reaktionskammer zusammen.

Die Fluidführung ist in Fließrichtung des fluiden Mediums dem Auslaufrohr vorgestellt. In einer Ausführungsform ist die Fluidführung zur Längsachse der Reaktionskammer geometrisch und spiegelsymmetrisch geformt.

In einer Ausführungsform ist die Fluidführung geometrisch flach zur Längsachse der Reaktionskammer geformt.

In einer bevorzugten Ausführungsform ist die Fluidführung zur Längsachse der Reaktionskammer geometrisch aufsteigend, bevorzugt länglich, geformt und weist einen rohrförmigen Zapfen, im Folgenden Ausstülpung der Fluidführung, kurz Ausstülpung, genannt, auf.

Die Länge der Ausstülpung ist variabel gestaltbar. Bevorzugt ragt die Ausstülpung in den Sohlebereich des unteren Teils der Reaktionskammer. Die Eintrittsöffnung des Auslaufrohres liegt mit der Ausstülpung der Fluidführung zentral auf der Längsachse der Reaktionskammer. In einer Ausführungsform ragt die Ausstülpung bis an die Eintrittsöffnung des Auslaufrohres.

In einer besonders bevorzugten Ausführungsform ist die Länge der Ausstülpung vorteilhaft so gestaltet, dass diese im engsten Teil der Düse, also der Stelle in der Düse mit dem kleinsten freien Querschnitt der fluidkontaktseitigen Innenwand des Auslaufrohres und somit der Düse zur Realisierung des Venturi-Effekts, endet. Vorteilhaft ist die strömungsdynamische Behandlung des fluiden Mediums durch diese Position optimiert.

In einer bevorzugten Ausführungsform ist die Fluidführung gegenüber dem Sohlebereich des Gehäuses und somit gegenüber dem Sohlebereich der Reaktionskammer entlang der Längsachse verschiebbar und verstellbar und passt sich somit vorteilhaft den Eigenschaften und der Behandlung des fluiden Mediums an.

Weiterhin ist der Abstand zwischen Fluidführung und Eintrittsöffnung des Auslaufrohres zentral entlang der Längsachse variabel einstellbar, sodass die Fluidführung bei Lageänderung des Auslaufrohres vorteilhaft entlang der Längsachse nachgeführt werden kann. Da auch das Auslaufrohr entlang der Längsachse variabel verstellbar ist, kann umgekehrt, das Auslaufrohr bei Lageänderung der Fluidführung, entlang der Längsachse nachgestellt werden.

Die Fluidführung ist bevorzugt in gleicher Weise wie das Auslaufrohr gegenüber ihrer Öffnung im Gehäuse entlang der Längsachse der Reaktionskammer verstellbar und verschiebbar, wodurch vorteilhaft die Druck- und Strömungsverhältnisse in der Reaktionskammer optimiert werden. Dazu ist der Verstellmechanismus der Fluidführung so gestaltet, dass deren Nachstellung oder-regelung in einen optimalen Unterdruckbereich für das Umlenken des durch die erfindungsgemäße Reaktoranlage erzeugten Fluidwirbels in das Auslaufrohr hinein ermöglicht wird.

Erfindungsgemäß wird der erzeugte Fluidwirbel an der Fluidführung in eine der in Einbaulage nach unten gerichteten Translations- und Rotationsbewegung entgegengesetzt aufsteigende Bewegung entlang der Längsachse durch eine Richtungsänderung in die Eintrittsöffnung des Auslaufrohrs umgelenkt.

Bei einem Anstellwinkel von *α* = 90° wird das fluide Medium im oberen Teil der Reaktionskammer in Rotation versetzt und rotiert entlang der Mittelebene zum Auslaufrohr. Der Drehimpuls des fluiden Mediums bleibt über den gesamten Bereich des oberen Teils der Reaktionskammer konstant und nimmt vorteilhaft erst beim Übergang des fluiden Mediums in den unteren Teil der Reaktionskammer durch die in Fließrichtung fallende Bewegung des fluiden Mediums ab.

Die Funktion der erfindungsgemäßen Reaktoranlage beruht auf der Initiierung physikalischer, mechanischer und chemischer Reaktionen durch Schaffung geeigneter Druckverhältnisse in der Reaktionskammer.

Die Stärke und damit die Wirksamkeit der erfindungsgemäßen Reaktoranlage sind druck-, geschwindigkeits- und temperaturabhängig. Die rotationssymmetrische Gestaltung der Reaktionskammer bewirkt in dem sich bildenden Fluidwirbel eine so starke Beschleunigung des Volumenstroms, dass die in dem fluiden Medium ablaufenden biologischen, physikalischen und chemischen Prozesse beschleunigt werden. Der Volumenstrom stellt sich dabei u.a. in Abhängigkeit von der Größe der Reaktionskammer bzw. dem Reaktionskammervolumen variabel ein.

Durch die Form der fluidführenden rotationssymmetrischen Reaktionskammer kommt es entlang der Längsachse zur Ausbildung von Translations- und Rotationsbewegungen des mindestens einen eingeführten Volumenstroms des fluiden Mediums, welcher einen Fluidwirbel ausbildet. Der Fluidwirbel wird dabei in Fließrichtung in Einbaulage zum unteren Ende der Reaktionskammer um das Auslaufrohr herum geführt und nimmt (im Falle von *α* > 90°) die Bewegung einer fallenden und in Fließrichtung nach unten gerichteten Schraubenlinie ein. Es kommt zur Bildung eines Fluidwirbels, welcher rotierend in den unteren Teil der Reaktionskammer geführt wird und eine Beschleunigung erfährt. Die Beschleunigung ist dabei hauptsächlich abhängig von den Parametern *r*₁, *b, r*₃, *z* und *a*.

Durch die Verjüngung des unteren Teils der Reaktionskammer im Längsschnitt der Reaktoranlage in Fließrichtung des fluiden Mediums wird der Fluidwirbel stark beschleunigt. Die kinetische Energie der Elementarteilchen im Fluidwirbel steigt aufgrund der Verjüngung des unteren Teils der Reaktionskammer und führt zum Anstieg der Reaktionsfähigkeit des fluiden Mediums.

Vorteilhaft kommt es zur Überlagerung von Translations- und Rotationsbewegung. Die Einströmgeschwindigkeit des mindestens einen fluiden Mediums ist so zu wählen, dass sich strömungstechnisch eine turbulente Grenzschicht ausbilden kann, der mindestens eine erzeugte Fluidwirbel beschleunigt wird und eine hohe Geschwindigkeitsdifferenz entsteht.

Vorzugsweise wird eine Kombination aus Translationsbewegung und gleichzeitiger Rotationsbewegung so gewählt, dass sich im Falle mehrerer Volumenströme diese berühren.

Die konstruktive Gestaltung der Erfindung ist so gewählt, dass dem fluiden Medium beim Durchfließen der Reaktionskammer unter definiertem Staudruck eine Geschwindigkeit mit einem möglichst hohen Maximalwert und einem möglichst großen Gradienten in radialer Richtung verliehen wird.

Die zur Erzeugung einer möglichst vorteilhaft starken Reibung und Zentrifugenwirkung und möglichst großer Schubspannungen in dem zu behandelnden Fluidwirbel benötigten Strömungsverhältnisse werden durch die konstruktive Gestaltung der Reaktoranlage erzielt. Durch die Form der Reaktionskammer wird der Fluidwirbel des zu behandelnden fluiden Mediums so gelenkt, dass sich im absteigenden Ast des Strömungsverlaufes, d.h. zwischen der mindestens einen Zuführungsöffnung mit sich anschließendem Fluideintrittsbereich und Eintrittsöffnung des Auslaufrohres, ein Fluidwirbel ausbildet. Die Strömungsgeschwindigkeit des Fluidwirbels weist über dessen Querschnitt in radialer Richtung einen starken Gradienten auf.

Durch die Verjüngung des unteren Teils der Reaktionskammer im Längsschnitt der Reaktoranlage in Fließrichtung des fluiden Mediums und durch die Anordnung der mindestens einen Zuführungsöffnung und der Eintrittsöffnung des Auslaufrohres werden zum einen Schubspannungen zwischen den einzelnen Strömungsschichten des Fluidwirbels, aber auch Schubspannungen zwischen den Wänden der Reaktionskammer, der Außenwand des in der Reaktionskammer befestigten Auslaufrohres und des Fluidwirbels erzeugt. Die durch die Schubspannungen erzeugten und diesen entgegengesetzten Reibungskräfte innerhalb des Fluidwirbels führen aufgrund einer Neuordnung der Bindungen zwischen den Molekülen des zu behandelnden fluiden Mediums zu einer Änderung der Oberflächenspannung und einer Veränderung der Viskosität des fluiden Mediums.

Es kann vorteilhaft eine Stofftrennung aufgrund der unterschiedlichen spezifischen Gewichte der im fluiden Medium befindlichen Stoffe erreicht werden, welche durch die Überlagerung von Translations- und Rotationsbewegung verstärkt wird.

Weiterhin wird eine Mahlwirkung erzielt. Die physikalisch erzeugte hohe Geschwindigkeitsdifferenz zwischen den einzelnen Schichten des Fluidwirbels führen zu einer mechanischen Zertrümmerung fester organischer Bestandteile, wie zum Beispiel Bakterien, Algen und anderer Mikroorganismen sowie anorganischer Inhaltsstoffe. Die entstehenden Trümmer werden in der Folge auf mechanischem und chemischem Wege abgebaut. Dieser mechanische Abbau der organischen und anorganischen Inhaltsstoffe findet in geringem Maße aufgrund der Geometrie der Reaktionskammer bereits vor dem Umlenken des gebildeten Fluidwirbels an der Fluidführung statt.

Zwischen oberem und unterem Teil der Reaktionskammer treten Druckdifferenzen auf, die vorteilhaft zur Erzeugung eines Fluidwirbels beitragen. Die sich einstellenden Drücke in der Reaktionskammer sind u.a. von der Gestaltung und Form der Reaktionskammer oder der Form der Düse abhängig. Im Sohlebereich vor der Eintrittsöffnung des Auslaufrohres herrschen ein Vordruck, welcher bevorzugt bei > ca. 3-4 bar liegt, ein in Fließrichtung zunehmender Staudruck sowie ein dadurch hervorgerufener Unterdruck bzw. Vakuum.

Durch die vorteilhafte Form des oberen Teils der Reaktionskammer wird im Vergleich zur EP 1 294 474 B2 weniger Druck und somit weniger Energie benötigt, um das durch die mindestens eine Zuführungsöffnung einströmende fluide Medium in Rotation zu versetzen. Andererseits kann durch die vorteilhafte Form des oberen Teils der Reaktionskammer bei im Vergleich zur EP 1 294 474 B2 gleichem benötigten Druck bzw. Energie eine höhere Drehzahl und Rotationsgeschwindigkeit des Fluidwirbels erreicht werden.

Die Wände der Reaktionskammer werden so bearbeitet, dass sie einen geringeren Reibungskoeffizienten als vor der Bearbeitung aufweisen und das fluide Medium so vorteilhaft in der Reaktionskammer beschleunigt werden kann. Der Reibungskoeffizient ist dabei abhängig vom entsprechend verwendeten Material der Reaktionskammer.

Durch die Gestaltung der erfindungsgemäßen Reaktoranlage wird das fluide Medium im oberen Teil der rotationssymmetrischen Reaktionskammer auf Basis des Drehimpulssatzes in Form eines geführten Fluidwirbels entlang der Längsachse in Fließrichtung in den unteren Teil der Reaktionskammer geführt, wobei es vorteilhaft und im Vergleich zur EP 1 294 474 B2 nur zu geringen Verlusten an Strömungsenergie kommt und sich der Drehimpuls des fluiden Mediums nur geringfügig ändert.

Im unteren Teil der Reaktionskammer wird der rotierende Fluidwirbel zum Zentrum der Strömung an der Fluidführung und dort in eine entgegengesetzte aufsteigende Richtung entlang der Längsachse der Reaktionskammer, bevorzugt in die Düse des Auslaufrohres umgelenkt. Bevorzugt wird der von oben kommende rotierende Fluidwirbel an der Fluidführung seiner Ursprungsrichtung entgegengesetzt umgelenkt. Dabei stößt der Fluidwirbel an die Fluidführung und es kommt zu einer Wirbeleinstülpung. Ganz besonders bevorzugt stößt der Fluidwirbel an die Ausstülpung der Fluidführung.

Die Zentrifugal- und Zentripetalkräfte und die durch Schubspannungen zwischen Strömungsschichten unterschiedlicher Geschwindigkeit hervorgerufene Reibungskräfte wirken im Sohlebereich des unteren Teils der Reaktionskammer sowie auf die im fluiden Medium enthaltenen unterschiedlich schweren Bestandteile unterschiedlich stark.

Im Sohlebereich kommt es zu einer starken Zentrifugenwirkung, weil die als Schwebeteilchen mitgeführten anorganischen und/oder organischen Verunreinigungen aufgrund ihres höheren Gewichtes vom Zentrum des Fluidwirbels zu seinem Rand getrieben werden, während gelöste gasförmige Bestandteile aufgrund ihres geringeren Gewichtes vom Rand des Fluidwirbels zu seinem Zentrum getrieben werden.

Bei der stattfindenden Richtungsänderung des Fluidwirbels durch die Umlenkung im Sohlebereich bewegen sich die schon getrennten Verunreinigungen und Medien unterschiedlicher Gewichte erneut über den über den Querschnitt des Fluidwirbels in die entgegengesetzte Richtung.

Somit arbeiten im unteren Bereich der Reaktionskammer, im Bereich vor der Eintrittsöffnung des Auslaufrohres, mindestens zwei Volumenströme gegeneinander (der Volumenstrom des in Einbaulage von oben kommenden Fluidwirbels und der Volumenstrom des umgelenkten Fluidwirbels). Es bildet sich vor der Eintrittsöffnung des Auslaufrohres ein niedrigerer Druck als im Rest der Reaktionskammer aus.

Durch die sich ausbildenden Druckverhältnisse im Sohlebereich der Reaktionskammer sowie vor der Eintrittsöffnung des Auslaufrohres werden die Zellwände der im fluiden Medium enthaltenen organischen Bestandteile zum Platzen gebracht. Weiterhin kommt es durch Kollision und Reibung der in dem fluiden Medium gelösten Fremdstoffe zur mechanischen und physikalischen Zerstörung und Zerkleinerung dieser Fremdstoffe. Zu den im fluiden Medium gelösten Fremdstoffen zählen organische und/oder anorganische Stoffe, Stoffverbindungen, Mikroorganismen sowie pflanzliche und/oder organische Lebewesen wie bspw. Keime, Bakterien, Pilze oder Algen untereinander sowie zu den einzelnen Teilchen, Atomen und Atomgruppen sowie Molekülen des fluiden Mediums.

Die hohe Bewegungsenergie, der Energieeintrag durch Reibung der einzelnen Schichten in dem mindestens einen Fluidwirbel, die damit verbundene hohe Zentrifugalkraft und/oder Translationskraft bewirken durch Aufbrechen und Neuformierung der vorhandenen Nebenvalenzverbindungen aufgrund deren unterschiedlicher Atommassen und damit unterschiedlicher Massenträgheit sowie durch Kollision der einzelnen Teilchen, Atome und Atomgruppen sowie Moleküle untereinander eine Neuordnung der Molekül- bzw. Gitterstruktur zu einem energetisch stabilen und ausgeglichenen, optimalen Zustand und Verbund und somit eine Änderung der im Normalfall vorliegenden Oberflächenspannung und Viskosität.

Das erfindungsgemäß behandelte fluide Medium behält seinen oberflächenentspannten Zustand über längere Zeit bei.

Durch die damit erreichte Neuordnung der Molekülstruktur werden in dem fluiden Medium gelöste Gase oder flüchtige Fremdstoffe freigesetzt, sodass zusätzlich eine Entgasung des fluiden Mediums stattfindet. Durch diese Entgasung werden zusätzlich unerwünschte Reaktionen dieser mitgeführten Stoffe im zu behandelnden fluiden Medium selbst, zu anderen mitgeführten Stoffen oder zu Stoffen, welche mit dem fluiden Medium in Berührung kommen, wie Messfühler oder Rohrwandungen, verringert oder verhindert.

Dabei ist die Zentrifugalkraft und/oder die Translationskraft so zu wählen, dass ein Aufbrechen der Stoffverbindungen und Molekülketten der im fluiden Medium gelösten Fremdstoffe stattfindet und diese mechanisch zerstört bzw. zerkleinert werden und/oder die vorhandenen Fremdstoffe oder die Atome, Moleküle oder Molekülverbindungen des fluiden Mediums mindestens teilweise ionisiert bzw. radikalisiert werden.

Durch die geometrische Gestaltung der rotationssymmetrischen Reaktionskammer werden die benötigten hohen und stark mit einem Gradienten behafteten Geschwindigkeiten im fluiden Medium erzeugt. Diese werden zur Erzielung der physikalischen Effekte, d.h. zur Zertrümmerung fester Bestandteile und zur Neuordnung molekularer Bindungen, und zur Auslösung und Beschleunigung der chemischen Prozesse durch Zufuhr von Energie benötigt. Die Quantität und Qualität der mechanischen Zerstörung und Zerkleinerung kann durch Änderung der Geschwindigkeiten je nach vorhandenem fluiden Medium und den darin gelösten Fremdstoffen eingestellt werden. Sie ist abhängig von der Widerstandsfähigkeit gegen mechanische Belastungen der Fremdstoffe.

Mitgeführte Stoffe werden aus der Gitterstruktur des fluiden Mediums gelöst und/oder über die Zentrifugalkraft aufgrund der unterschiedlichen spezifischen Stoffgewichte vom fluiden Medium getrennt und können im Anschluss beim Herausführen aus der Reaktionskammer durch das Auslaufrohr herausgefiltert, sedimentiert oder anderweitig gebunden werden. Durch die Zerkleinerung von Stoffen kann sich die elektrische Leitfähigkeit des fluiden Mediums erhöhen.

Durch die Gestaltung des Auslaufrohres im unteren mündungsnahen Bereich als Düse zur Realisierung des Venturi-Effekts in Verbindung mit dem der Reaktionskammer zugeführten fluiden Medium wird der Fluidwirbel, welcher sich als Hohlwirbel ausbildet, im Auslaufrohr stark beschleunigt und entspannt, wodurch bei flüssigen fluiden Medien der Dampfdruck im Kernbereich erreicht oder unterschritten werden kann, und eine Strömung mit im Kern- und Randbereich stark unterschiedlichen Geschwindigkeiten entsteht.

Es entsteht ein Hohlwirbel, in dessen Zentrum sich ein Kern aus einem leichteren fluiden Medium als im übrigen Strömungsfeld bildet. Bei wachsenden Geschwindigkeiten werden Wirbelströmungen mit Wirbelfaden oder Wirbelröhren oder, je nach Art des fluiden Mediums, ein drehungsfreier Fluidwirbel mit Wirbelkern, auch als Potentialwirbel bekannt, erzeugt. Dabei werden nochmals Schubspannungen im fließenden fluiden Medium erreicht, die die physikalischen und chemischen Prozesse weiter befördern.

Dieser Hohlwirbel mit Wirbelkern, welcher ein Vakuum bildet, überlagert sich mit der entstehenden Vakuumbildung in der Düse mit Venturi-Effekt aufgrund des Venturi-Effektes.

Durch die überlagerte und verstärkte Vakuumbildung im Düsenbereich werden Keime und Bakterien, welche einen inneren Zelldruck (Turgor) besitzen, aufgerissen und oxidiert. Im Vakuumbereich werden gelöste Gase in einem flüssigen fluiden Medium aufgrund des vorliegenden Fluidwirbels gelöst und entgast.

Wird im Gegenzug über die Fluidführung, welche im Zentrum entlang der Längsachse eine im Durchfluss einstellbare Durchgangsbohrung besitzen kann, der Reaktionskammer ein Gas als zusätzliches fluides Medium zugeführt, so vermischt es sich mit dem Fluidwirbel und wird aufgrund der geänderten Molekülstruktur des fluiden Mediums deutlich besser im fluiden Medium gelöst.

Besondere Vorteile der erfindungsgemäßen Vorrichtung und des erfindungsgemäßen Verfahrens bestehen darin, dass damit bei geringem Platz- und Kostenaufwand ohne Zugabe umweltschädlicher Chemikalien und ohne Bestrahlung des fluiden Mediums oder sonstiger potentiell gefährlicher Maßnahmen ein effektiver, kostengünstiger Prozess durchgeführt werden kann, in dessen Ergebnis nach Anwendungszweck Abwässer entgiftet und entkeimt wieder der Nutzung zugeführt werden können, Wasserreservoirs keimfrei gehalten werden können, in Gebieten mit Wasserknappheit eine Versorgung mit Frischwasser sichergestellt werden kann, die Benetzungsfähigkeit verschiedener Flüssigkeiten erhöht werden kann, die Verwendung waschaktiver Chemikalien zu verschiedensten Reinigungszwecken in Haushalt und Industrie signifikant vermindert und so die Umweltbelastung reduziert werden kann oder dickflüssige Medien ohne chemische Veränderung auf rein mechanischem Wege verdünnt werden können.

Durch die erfindungsgemäße Gestaltung der Reaktoranlage bzw. des oberen Teils der Reaktionskammer erreicht der Fluidwirbel eine erhöhte Rotationsgeschwindigkeit, wodurch die Effizienz der Zerstörung und Zerkleinerung der Fremdstoffe erheblich und vorteilhaft gesteigert wird. Durch die erfindungsgemäße Gestaltung der Reaktionskammer, speziell des oberen Teils der Reaktionskammer, ist dabei bis zur Richtungsänderung des erzeugten Fluidwirbels durch Umlenkung an der Fluidführung entlang der Längsachse durch die Rotationsgeschwindigkeit der Dampfdiffusionsdruck noch nicht erreicht. Dadurch ist vorteilhaft eine Energieeinsparung durch eine Druckreduzierung von bis zu 50 %, bevorzugt von 20-40 %, ganz besonders bevorzugt von 20-30 % möglich.

Es ist zu beachten, dass sich die der Reaktionskammer zugeführten fluiden Medien hinsichtlich ihrer Eigenschaften wie bspw. Oberflächenspannung oder Viskosität unterscheiden und somit jeweils andere chemische Reaktionen und Messparameter in der erfindungsgemäßen Reaktoranlage hervorrufen. Die Messparameter variieren somit je nach den eingesetzten fluiden Medien.

Vorteilhaft kann ein Katalysator zur Beschleunigung der chemischen Reaktionen in der Reaktoranlage hinzugefügt werden. In einer besonderen Ausführung ist zumindest ein Teil der fluidführenden Wände der Reaktionskammer katalytisch beschichtet oder die fluidführenden Wände der Reaktionskammer bestehen komplett aus einem katalytischen Material.

Weiterhin können die chemischen Reaktionen durch eine Temperaturerhöhung der fluiden Medien entsprechend der thermischen Zustandsgleichung idealer Gase beschleunigt werden. Vorteilhaft ist die Reaktionsgeschwindigkeit durch den durch Temperaturerhöhung höheren Energieeintrag höher. Dazu können bereits erwärmte fluide Medien wie bspw. warme oder heiße Abwässer der Reaktoranlage zugeführt und strömungsdynamisch behandelt werden. In einer alternativen Ausführung ist die Reaktoranlage mit einer Heizung, bspw. einer Heizplatte, und dazugehöriger Heizsteuerung zur Erwärmung der fluiden Medien verbunden.

Weiterhin sind Peripheriebauteile wie Schläuche oder Rohre zum Transport des fluiden Mediums, Druckventile wie bspw. Überdruckventile, Strömungsteiler und Vorbehandlungseinheiten an die erfindungsgemäße Reaktoranlage anschließbar. Die Verwendung von Pumpen und/oder Kompressoren erzeugt in Verbindung mit der Verstellbarkeit des Auslaufrohres sowie des freien Querschnitts der Eintrittsöffnung des Auslaufrohres den benötigten Staudruck.

In einer Ausführungsform ist eine Einrichtung zur Messung des pH-Wertes an die Reaktoranlage angeschlossen. In einer Ausführungsform wird die Reaktoranlage in einem offenen Rohrleitungssystem verwendet. Somit lässt sich vorteilhaft der pH-Wert des fluiden Mediums nach der strömungsdynamischen Behandlung messen.

Weiterhin werden die während der strömungsdynamischen Behandlung des fluiden Mediums entstehenden Gase mit dem fluiden Medium durch die Rotationsbewegung aus der Austrittsöffnung des Auslaufrohres aus der Reaktoranlage abtransportiert und neutralisiert.

In einer alternativen Ausführungsform wird die Reaktoranlage in einem geschlossenen Zirkulationssystem verwendet. Die während der strömungsdynamischen Behandlung entstehenden Gase werden mit dem fluiden Medium durch Rotationsbewegung aus der Austrittsöffnung des Auslaufrohres abtransportiert und neutralisiert. In einer Ausführungsform werden die entstandenen abgeführten Gase in einer Einrichtung zum separierten Auffangen gesammelt. Vorzugsweise sind dies spezielle Auffangbehälter für Gase. Vorteilhaft lässt sich eine Knallgasreaktion durch das separate Auffangen sowie eine anschließende Neutralisation vermeiden.

In einer besonderen Ausführungsform werden die aufgefangenen Gase weiterverwendet und bspw. vorteilhaft für Brenn- oder Heizstoffe wie Methan, Methanol oder Benzol weiter verwertet.

In einer bevorzugten Ausgestaltung weist die Fluidführung mindestens eine Durchgangsbohrung entlang der Längsachse auf, wobei die Längsachse der Durchgangsbohrung mit der Längsachse der rotationssymmetrischen Reaktionskammer zusammen fällt.

Durch die Durchgangsbohrung entlang der Längsachse der Fluidführung kann der Reaktionskammer vorteilhaft bevorzugt mindestens ein zusätzliches fluides Medium zugeführt werden, welches durch den im Sohlebereich der Reaktionskammer herrschenden Unterdruck bei Bedarf direkt und selbsttätig in den unteren Teil der Reaktionskammer angesaugt wird. Vorteilhaft wird der Fluidwirbel somit an der Fluidführung umgelenkt und kann zusätzlich mit einem zusätzlichen fluiden Medium versetzt werden.

In einer Ausführungsform ist die Fluidführung zur Längsachse der Reaktionskammer geometrisch flach geformt und weist eine Durchgangsbohrung auf. In einer bevorzugten Ausführungsform ist die Fluidführung zur Längsachse der Reaktionskammer geometrisch aufsteigend, bevorzugt länglich, geformt und weist einen rohrförmigen Zapfen, im Folgenden Ausstülpung der Fluidführung, kurz Ausstülpung, genannt, auf, welcher eine Durchgangsbohrung besitzt.

Ist die Länge der Ausstülpung der Fluidführung so geformt, dass sie direkt in der Düse zur Realisierung des Venturi-Effekts endet, wird das durch die Durchgangsbohrung zusätzlich angesaugte fluide Medium somit direkt in das Innere des in Einbaulage unteren mündungsnahen Bereichs des Auslaufrohres gesaugt.

Vorteilhaft an der länglichen Ausgestaltung der Fluidführung mit der Durchgangsbohrung entlang der Längsachse ist das gezielte Zuführen bzw. Ansaugen eines zusätzlichen fluiden Mediums direkt durch die Eintrittsöffnung des Auslaufrohres in dessen unteren mündungsnahen Bereich. Bevorzugt ist der untere mündungsnahe Bereich des Auslaufrohres als Düse zur Realisierung des Venturi-Effekts ausgebildet, wodurch das zusätzliche fluide Medium direkt in die Düse zur Realisierung des Venturi-Effekts angesaugt wird.

Dabei ist die Länge der Ausstülpung variabel gestaltbar. In einer besonders bevorzugten Ausführungsform und für die höchste Effizienz der Reaktionen ist die Länge der Ausstülpung vorteilhaft so gestaltet, dass diese im engsten Teil der Düse, also der Stelle in der Düse mit dem kleinsten freien Querschnitt der fluidkontaktseitigen Innenwände des Auslaufrohres, endet. Vorteilhaft ist die strömungsdynamische Behandlung des fluiden Mediums an dieser Position optimiert.

Die Medienzugabe für chemische Nachreaktionen im Auslaufrohr erfolgt durch Druck oder vorteilhaft durch Nutzung des Unterdrucks in der Düse zur Realisierung des Venturi-Effekts.

In einer Ausführungsform wird der Reaktionskammer ein zusätzliches fluides Medium zugeführt. Dieses kann durch die Durchgangsbohrung der Fluidführung angesaugt werden oder auch über den Hauptzufluss oder andere Zuleitungen über die mindestens eine Zuführungsöffnung in den sich anschließenden Fluideintrittsbereich des oberen Teils der Reaktionskammer gelangen.

In einer Ausführungsform werden der Reaktionskammer mehrere zusätzliche fluide Medien zugeführt. Diese können alle durch die Durchgangsbohrung der Fluidführung angesaugt werden oder auch über den Hauptzufluss oder andere Zuleitungen über die mindestens eine Zuführungsöffnung in die Reaktionskammer gelangen. In einer weiteren Ausführungsform gelangen die fluiden Medien durch die Fluidführung sowie über den Hauptzufluss oder andere Zuleitungen über die mindestens eine Zuführungsöffnung in die Reaktionskammer. Auch im zusätzlichen fluiden Medium gelöste Feststoffe können durch die Durchgangsbohrung und/oder die mindestens eine Zuführungsöffnung in die Reaktionskammer angesaugt werden.

In einer Ausführungsform kann das mindestens eine zusätzliche fluide Medium dasselbe Medium sein, was der Reaktoranlage durch die mindestens eine Zuführungsöffnung im oberen Teil der Reaktionskammer zugeführt wird. In einer alternativen Ausführungsform ist das mindestens eine zusätzliche fluide Medium ein anderes fluides Medium als jenes, was der Reaktoranlage durch die mindestens eine Zuführungsöffnung im oberen Teil der Reaktionskammer zugeführt wird. Dadurch ist eine gezielte Dosierung von weiteren zusätzlichen fluiden Medien möglich.

Durch die zusätzlich zugeführten fluiden Medien lassen sich bevorzugt chemische oder biologische Reaktionen verbessern bzw. beschleunigen, indem chemische oder biologische Reaktionen beeinflussende Stoffe wie beispielweise Oxidations- oder Fällungsmittel zur Reaktion gebracht werden. Als zusätzlich zugeführte fluide Medien kommen bspw. Oxidationsmittel wie Ozon, Wasserstoffperoxid oder Sauerstoff oder andere als Reaktionspartner und Katalysatoren dienende zusätzliche fluide Medien in Frage, welche der Reaktionskammer aus einem Reservoir zugeführt werden.

Ist das zusätzliche zugeführte fluide Medium gasförmig und ein Oxidationsmittel, wie bspw. Sauerstoff oder Sauerstoff aus der (Umgebungs-)Luft, so kann dieses zur Verbesserung der Oxidationseigenschaften durch eine vorgeschaltete Vorbehandlungseinrichtung ionisiert bzw. zu Radikalen wie bspw. Ozon umgewandelt werden. Dadurch können Kohlenwasserstoffverbindungen und/oder andere organische Verbindungen wie Keime, Bakterien und Kleinstlebewesen oxidiert werden. Dabei entsteht u.a. Wasser und Kohlendioxid, d.h. bei organischen Substanzen findet eine Denaturierung statt.

Durch die dosierte Einspeisung von Oxidationsmitteln oder anderen als Reaktionspartnern dienenden zusätzlichen fluiden Medien wird eine signifikante Steigerung der Reaktionsgeschwindigkeit hervorgerufen.

Wichtigste Einsatzgebiete des erfindungsgemäßen Verfahrens bzw. die mit der erfindungsgemäßen Vorrichtung behandelten fluider Medien sind Industrie, Gewerbe, private Haushalte, Lebensmittelherstellung, Land- und Forstwirtschaft, Abfall- und Entsorgungswirtschaft, Reinigungstechnik, Entkeimung, Konservierung, Maschinenbau, Elektronik, Medizin und Therapie, Bauindustrie sowie Energietechnik.
Die erfindungsgemäße Vorrichtung und das erfindungsgemäße Verfahren werden bevorzugt zur Vorbehandlung, Aufbereitung, Entkeimung, Desinfektion und/oder Initiierung mechanischer, physikalischer und chemischer Reaktionen von und in fluiden Medien verwendet. Bevorzugt handelt es sich dabei um wässrige fluide Medien.

Erfindungsgemäß wird unter der Vorbehandlung, Aufbereitung, Entkeimung, Desinfektion und/oder Initiierung mechanischer, physikalischer und chemischer Reaktionen die Reinigung und Säuberung von fluiden Medien verstanden, wobei der Anteil an Schadstoffen reduziert wird. Schadstoffe sind im fluiden Medium gelöste organische oder anorganische Bestandteile oder Mikroorganismen, welche auch giftig sein können.

So werden bspw. in wässrigen Lösungen befindliche Kohlenwasserstoffe, Keime, Pilze, Algen und Bakterien zerstört, indem organische Bestandteile zum Platzen gebracht und schwer lösliche und giftige anorganische Bestandteile dabei zerstört werden. Besonders bevorzugt wird Trinkwasser, Brauchwasser, Prozesswasser, Abwasser oder Grauwasser vorbehandelt, aufbereitet und/oder desinfiziert. Auch langkettige molekulare Verbindungen können zerkleinert werden.

Beispielweise wird damit das Wasser in Schwimmbädern desinfiziert. Vorteilhaft kann die erfindungsgemäße Vorrichtung und das erfindungsgemäße Verfahren in der autarken Versorgung von Trinkwasser, aber auch in der (mobilen) Aufbereitung von Abwasser in Wohnmobilen sowie in der Aufbereitung von Abwasser in entlegenen Bergdörfern oder autarken Feriensiedlungen angewendet werden.

Die erfindungsgemäße Vorrichtung und das erfindungsgemäße Verfahren werden bevorzugt zur Behandlung Abwässern, bevorzugt von privaten, industriellen oder kommunalen Abwässern eingesetzt. Beispielweise werden dort gelöste Kohlenwasserstoffverbindungen zumindest angeknackt und dann von anderen Bakterien gefressen. Weiterhin können mit der erfindungsgemäßen Vorrichtung und dem erfindungsgemäßen Verfahren chemisch belastete Gewässer gereinigt werden. Auch industriell hergestellte Seifenwässer werden so gereinigt.

Auch mineralölhaltige Abwässer wie sie bspw. bei Tankstellen, (Auto-)Waschanlagen, Industriewaschanlagen sowie organisch hochbelasteten Abwässer wie bspw. bei Biogasanlagen anfallen, werden mit der erfindungsgemäßen Vorrichtung und dem erfindungsgemäßen Verfahren vorteilhaft aufgereinigt. Weiterhin können damit in Abwässern vorkommende Tenside aufgereinigt und aufbereitet werden.

Weiterhin können mit der erfindungsgemäßen Vorrichtung und dem erfindungsgemäßen Verfahren auch Schmierölemulsionen sowie Schweröle aufgereinigt werden.

Werden Teilen der Autokarosserie geformt, müssen die mit Fett eingestrichenen Bleche vor dem Lackieren wieder mit heißem Wasser gereinigt werden. Das Reinigungswasser muss ebenfalls von Fetten und Tensiden gereinigt werden. Diese Aufbereitung des Rückstandswassers, welches bei der Formgebung von Metallkarosserien nach der Metallwäsche entsteht, kann durch die erfindungsgemäße Vorrichtung und das erfindungsgemäße Verfahren ebenfalls realisiert werden.

Weiterhin können gasförmige oder flüssige Kraftstoffe welche bevorzugt auf pflanzlichen Ölen basieren, behandelt werden.

Besondere Vorteile der erfindungsgemäßen Vorrichtung und des erfindungsgemäßen Verfahrens bestehen darin, dass damit bei geringem Platz- und Kostenaufwand ohne Zugabe umweltschädlicher Chemikalien und ohne Bestrahlung des fluiden Mediums oder sonstiger potentiell gefährliche Maßnahmen ein effektiver, kostengünstiger Prozess durchgeführt werden kann, in dessen Ergebnis nach Anwendungszweck Abwässer entgiftet und entkeimt wieder der Nutzung zugeführt werden können, Wasserreservoirs keimfrei gehalten werden können, in Gebieten mit Wasserknappheit eine Versorgung mit Frischwasser sichergestellt werden kann, die Benetzungsfähigkeit verschiedener Flüssigkeiten erhöht werden kann, die Verwendung waschaktiver Chemikalien zu verschiedensten Reinigungszwecken in Haushalt und Industrie signifikant vermindert und so die Umweltbelastung reduziert werden kann oder dickflüssige Medien ohne chemische Veränderung auf rein mechanischem Wege verdünnt werden können.

### Ausführungsbeispiele

Nachfolgend soll die Erfindung anhand einiger Ausführungsbeispiele eingehender erläutert werden. Die Ausführungsbeispiele sollen dabei die Erfindung beschreiben ohne diese zu beschränken.

Anhand von Zeichnungen wird die Erfindung näher erläutert. Dabei zeigen
- Fig. 1: Draufsicht im Querschnitt entlang der Schnittebene C-C auf die erfindungsgemäße Reaktoranlage,
- Fig.2: die erfindungsgemäße Reaktoranlage bei einem Anstellwinkel von *α* = 90° im Längsschnitt der Reaktoranlage entlang der Schnittebene D-D,
- Fig.3: die erfindungsgemäße Reaktoranlage bei einem Anstellwinkel von *α* = 90° und einem sich verringernden Abstand zwischen Deck- und Grundfläche des oberen Teils der Reaktionskammer im Längsschnitt der Reaktoranlage,
- Fig. 4: eine weitere Draufsicht im Querschnitt entlang der Schnittebene B-B auf die erfindungsgemäße Reaktoranlage,
- Fig. 5: ein Sprengbild der erfindungsgemäßen Reaktoranlage bei einem Anstellwinkel von *α* = 90° im Längsschnitt der Reaktoranlage entlang der Schnittebene A-A,
- Fig. 6: die erfindungsgemäße Reaktoranlage bei einem Anstellwinkel von *α* = 90° und einem sich verringerndem Abstand zwischen Deck- und Grundfläche des oberen Teils der Reaktionskammer im Längsschnitt der Reaktoranlage,
- Fig. 7: die erfindungsgemäße Reaktoranlage bei einem Anstellwinkel von *α* = 110° im Längsschnitt der Reaktoranlage.

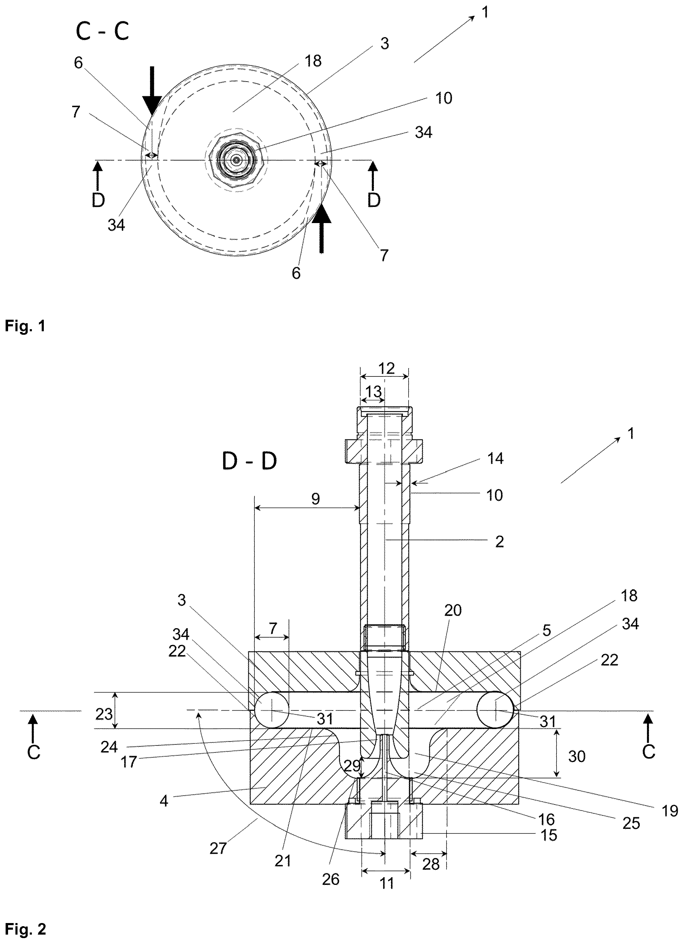

Figur 1 zeigt in der Draufsicht im Querschnitt entlang der Ebene C-C die erfindungsgemäße Reaktoranlage 1 mit dem oberen Teil des Gehäuses 3 und dem Auslaufrohr 10. Zwei sich im Längsschnitt der Reaktoranlage 1 entlang der Ebene D-D gegenüberliegende Zulaufrohre (nicht gezeigt) münden in der Ansicht entlang der Ebene C-C tangential zur Mantelfläche des oberen Teils der Reaktionskammer 18 und bilden zwei Zuführungsöffnungen 6, welche im Schnitt mit der Mantelfläche eine elliptische Fläche aufweisen. Die beiden Pfeile vor den jeweiligen Zuführungsöffnungen 6 stellen die Fließrichtung des fluiden Mediums dar. An die Zuführungsöffnungen 6 schließen sich jeweils die Fluideintrittsbereiche 34 in Fließrichtung an, welche jeweils im Längsschnitt zur Reaktoranlage 1 eine kreisförmige Fläche mit einem Durchmesser *d*_{z} 7 aufweisen.

Figur 2 zeigt den Aufbau der erfindungsgemäßen Reaktoranlage 1 aus dem Querschnitt entlang der Ebene C-C von Figur 1 im Längsschnitt der Reaktoranlage 1 entlang der Ebene D-D, wobei die Komponenten bzw. Teile der Reaktoranlage 1 entlang der Längsachse 2 angeordnet sind. Der Längsschnitt der Reaktoranlage 1 entlang der Ebene D-D verläuft so, dass der Fluideintrittsbereich 34 auf der in der Schnittdarstellung in Einbaulage linken und rechten Seite dargestellt ist. Das eingeleitete fluide Medium strömt auf der linken Seite aus der Schnittebene D-D heraus. Bei dem Fluideintrittsbereich 34 auf der in der Schnittdarstellung rechten Seite wiederum strömt das eingeleitete fluide Medium in die Schnittebene D-D hinein. Alle Merkmale bzw. Bezugszeichen beziehen sich auf eine Hälfte der Reaktoranlage 1 im Längsschnitt. Der Aufbau der zweiten Hälfte der Reaktoranlage 1 auf der anderen Seite der Längsachse 2 ist jedoch derselbe, da die Reaktoranlage 1 im Längsschnitt spiegelsymmetrisch aufgebaut ist.

Die Reaktoranlage 1 ist entlang der Mittelebene 5 in einen oberen Teil 3 und einen unteren Teil 4 des Gehäuses unterteilt, wobei die fluidkontaktseitigen Innenwände des Gehäuses 3, 4 eine rotationssymmetrische Reaktionskammer 18, 19 formen, welche ebenfalls einen oberen Teil 18 und einen unteren Teil 19 aufweist. Der obere Teil der Reaktionskammer 18 weist eine Deckfläche 20 und eine Grundfläche 21 sowie einen Übergangsbereich von der Deck- zur Grundfläche 22 auf. Die Längsachse 2 entspricht der Drehachse der rotationssymmetrischen Reaktionskammer 18, 19. Weiterhin befindet sich ein Auslaufrohr 10 in der Reaktoranlage 1.

Das fluide Medium wird durch eine Zuführungsöffnung (im Längsschnitt nicht gezeigt), welche tangential im Querschnitt zur Mantelfläche des oberen Teils der Reaktionskammer 18 angeordnet ist, in den oberen Teil der Reaktionskammer 18 eingeleitet. An die Zuführungsöffnung (im Längsschnitt nicht gezeigt) schließt sich in Fließrichtung ein Fluideintrittsbereich 34 an, welcher im Längsschnitt zur Reaktoranlage 1 eine an der Längsschnittkante kreisförmige Fläche mit einem Durchmesser *d*_{z} 7 und einen dazugehörigen Mittelpunkt 31 aufweist. Die Mittelebene 5 verläuft durch den Mittelpunkt 31 des Fluideintrittsbereiches 34. Der Abstand *b* 23 zwischen Deckfläche 20 und Grundfläche 21 ist konstant. Der Anstellwinkel *α* 27 beträgt 90° und bezieht sich auf den Winkel, welcher sich, im Längsschnitt in Einbaulage gesehen, von der Mittelebene 5, die durch die Mittelpunkte 31 des Fluideintrittsbereiches 34 verläuft, zur Längsachse 2 einstellt. Der Anstellwinkel 27 bei *α* = 90° bezieht sich dabei auf den sich in Einbaulage einstellenden Winkel unterhalb der Mittelebene 5, d.h. von der Mittelebene 5 zur Längsachse 2 der Reaktionskammer 18, 19. Dazu stellt der Schnitt der Längsachse 2 mit der Mittelebene 5 ein kartesisches Koordinatensystem dar. Der Anstellwinkel *α* 27 = 90° bezieht sich somit immer auf den dritten und vierten Quadranten des kartesischen Koordinatensystems. Bei dem Anstellwinkel *α* 27 = 90° ist der Abstand *b* 23 gleich dem Durchmesser *d*_{z} 7 des Fluideintrittsbereiches 34 und entspricht somit der Höhe des oberen Teils der Reaktionskammer 18.

Der Abstand vom Übergangsbereich von der Deck- zur Grundfläche 22 im oberen Teil der Reaktionskammer 18 zur Außenwand des Auslaufrohres 10 entspricht dem maximalen Abstand *r*ₘₐₓ 9 des oberen Teils der Reaktionskammer 18. Die fluidführenden Wände der Reaktionskammer 18, 19 sind so beschaffen, dass sie hinsichtlich ihrer Geometrie und der Oberfläche einen geringen Reibungswiderstand und Reibungsbeiwert erzeugen.

Das fluide Medium wird im oberen Teil der Reaktionskammer 18 in Rotation versetzt und bildet einen Fluidwirbel, welcher in Fließrichtung entlang der Längsachse 2 den unteren Teil der Reaktionskammer 19 gelenkt wird. Der untere Teil der Reaktionskammer 19 erstreckt sich vom Übergang der Grundfläche 24 bis zu einem gekrümmten Sohlebereich 25 mit der unteren Begrenzung des Sohlebereichs 26. Dabei entspricht der Radius *r*₃ 28 dem Abstand vom Übergang der Grundfläche 24 des unteren Teils der Reaktionskammer 19 zur Außenwand des Auslaufrohres 10. Weiterhin entspricht *z* 30 dem Abstand des unteren Teils der Reaktionskammer 19 ab jener Stelle, an welcher die Deckfläche 20 und die Grundfläche 21 des oberen Teils der Reaktionskammer 18 keinen konstanten Abstand *b* 23 mehr zueinander aufweisen, wobei sich z 30 bis zur unteren Begrenzung 26 des Sohlebereichs 25 des unteren Teils der Reaktionskammer 19 erstreckt. Im unteren Teil des Gehäuses 4 ist eine geometrisch aufsteigend geformte Fluidführung 15 angeordnet, deren Längsachse mit der Längsachse 2 der Reaktionskammer 18, 19 zusammen fällt. Die Fluidführung weist eine Ausstülpung 16 mit einer Durchgangsbohrung auf, welche in die Stelle mit dem kleinsten freien Querschnitt der fluidkontaktseitigen Innenwände des Auslaufrohres 10 ragt. Durch die Durchgangsbohrung können zusätzliche fluide Medien bei Bedarf in den Sohlebereich 25 des unteren Teils der Reaktionskammer 19 angesaugt werden. Die Stelle mit dem kleinsten freien Querschnitt der fluidkontaktseitigen Innenwände des Auslaufrohres 10 ist als Düse zur Realisierung des Venturi-Effekts 17 ausgestaltet. Der rotierende Fluidwirbel wird unter Beibehaltung seiner Geschwindigkeit an der Fluidführung 15 umgelenkt und tritt durch die Eintrittsöffnung 11 des Auslaufrohres 10 in das Auslaufrohr 10 ein. Die Eintrittsöffnung 11 ist im unteren Teil der Reaktionskammer 19 angeordnet und in einem variablen Abstand *a* 29 von der unteren Begrenzung 26 des gekrümmten Sohlebereichs 25 des unteren Teils der Reaktionskammer 19 beabstandet. Weiterhin weist das Auslaufrohr einen Radius *r*₂ 13 von der Längsachse 2 zur Außenwand des Auslaufrohres 10 sowie eine Wanddicke d 14 auf. Das fluide Medium wird aus der Austrittsöffnung 12 des Auslaufrohres 10 aus der Reaktoranlage 1 herausgeleitet.

Die fluidführenden Wände der Reaktionskammer 18, 19 sind so beschaffen, dass sie hinsichtlich ihrer Geometrie und der Oberfläche einen geringen Reibungswiderstand und Reibungsbeiwert erzeugen. Der benötigte Druck zur Erzeugung des Fluidwirbels und der Realisierung des Venturi-Effekts in der Düse 17 mit einem überlagerten Unterdruck von ca. -0,99 bar liegt aufgrund der geringeren Fluidreibung in der erfindungsgemäßen Reaktionskammer 18, 19 mit 3,5 bar gegenüber der EP 1 294 474, welche bei gleichem Reaktionskammervolumen ein Druck von 6,0 bar benötigt, vorteilhaft um ca. 42% niedriger.

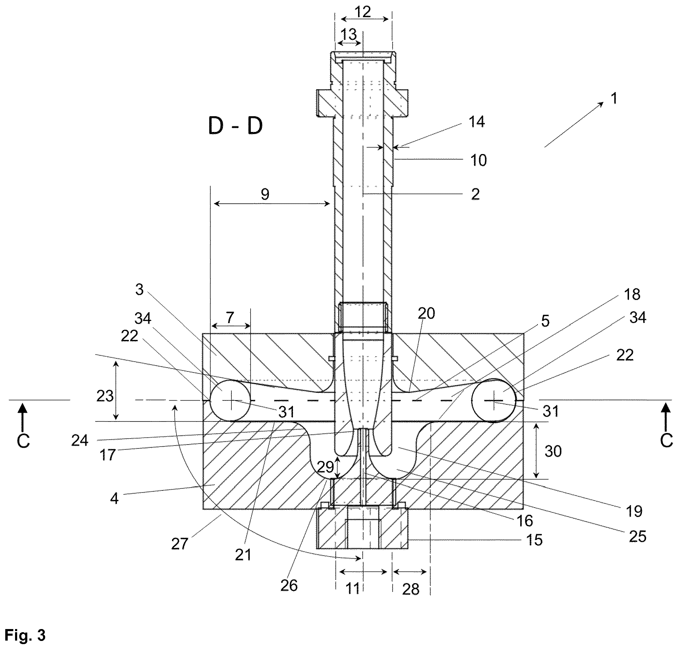

Figur 3 zeigt den Aufbau der erfindungsgemäßen Reaktoranlage 1 aus dem Querschnitt entlang der Ebene C-C von Figur 1 im Längsschnitt der Reaktoranlage 1 entlang der Ebene D-D, wobei die Komponenten bzw. Teile der Reaktoranlage 1 entlang der Längsachse 2 angeordnet sind. Der Großteil der Merkmale des Aufbaus entspricht jenen aus der Draufsicht des Querschnitts aus Figur 1 bzw. 3, weshalb auf diese nicht weiter eingegangen wird.

Der Anstellwinkel *α* 27 zur Längsachse 2 beträgt wiederum *α* = 90° und bezieht sich auf jenen Winkel, welcher sich, im Längsschnitt in Einbaulage gesehen, von der Mittelebene 5, die durch die Mittelpunkte 31 des Fluideintrittsbereiches 34 verläuft, zur Längsachse 2 einstellt. Der Abstand *b* 23 zwischen Deckfläche 20 und Grundfläche 21 ist im Bereich der Zuführungsöffnung (im Längsschnitt nicht gezeigt) und des Fluideintrittsbereiches 34 maximal (*b*ₘₐₓ) und entspricht dem kreisförmigen Durchmesser *d*_{z} 7 des Fluideintrittsbereiches 34. In Fließrichtung des fluiden Mediums verringert sich der Abstand *b* 23 zwischen Deckfläche 20 und Grundfläche 21 zur Außenwand des Auslaufrohres 10 hin, wodurch vorteilhaft eine zusätzliche Beschleunigung des fluiden Mediums erreicht wird.

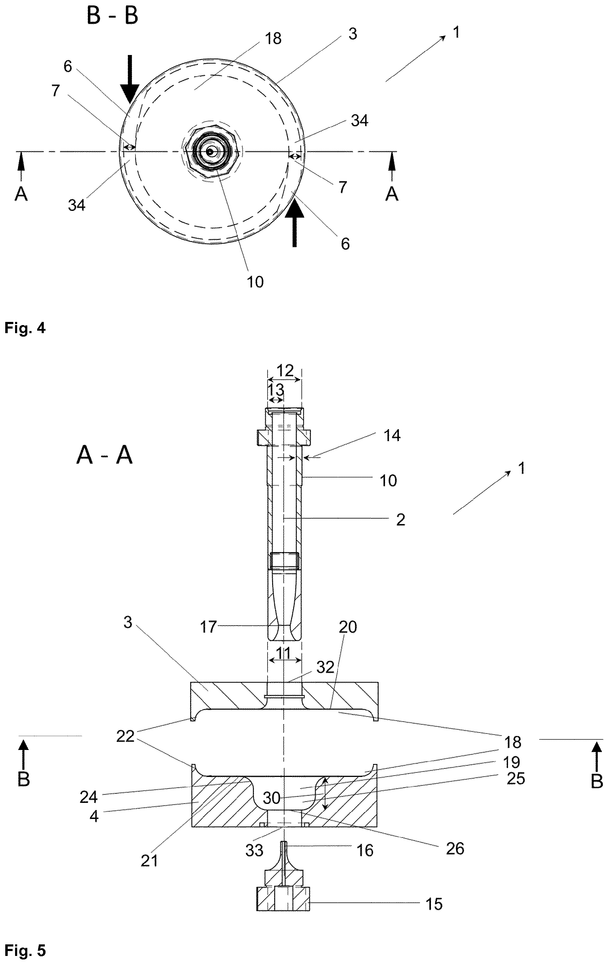

Figur 4 zeigt in der Draufsicht im Querschnitt entlang der Ebene B-B die erfindungsgemäße Reaktoranlage 1 mit dem oberen Teil des Gehäuses 3 und dem Auslaufrohr 10. Zwei sich im Längsschnitt der Reaktoranlage 1 entlang der Ebene A-A gegenüberliegende Zulaufrohre (nicht gezeigt) münden in der Ansicht entlang der Ebene B-B tangential zur Mantelfläche des oberen Teils der Reaktionskammer 18 und bilden zwei Zuführungsöffnungen 6, welche im Schnitt mit der Mantelfläche eine elliptische Fläche aufweisen. Die beiden Pfeile vor den jeweiligen Zuführungsöffnungen 6 stellen die Fließrichtung des fluiden Mediums dar. An die Zuführungsöffnungen 6 schließen sich jeweils die Fluideintrittsbereiche 34 in Fließrichtung an, welche jeweils im Längsschnitt zur Reaktoranlage 1 eine kreisförmige Fläche mit einem Durchmesser *d*_{z} 7 aufweisen.

Figur 5 zeigt im Sprengbild die Komponenten bzw. Teile der erfindungsgemäße Reaktoranlage 1 aus dem Querschnitt entlang der Ebene B-B von Figur 3, welche alle entlang der Längsachse 2 angeordnet sind. Im Längsschnitt der Reaktoranlage 1 entlang der Ebene A-A in Einbaulage sind gezeigt das Auslaufrohr 10, der obere Teil des Gehäuses 3 mit dem oberen Teil der Reaktionskammer 18, der untere Teil des Gehäuses 4 mit dem unteren Teil der Reaktionskammer 19 und die Fluidführung 15 mit der Ausstülpung 16. Weiterhin ist im oberen Teil des Gehäuses 3 die Öffnung 32 für das Auslaufrohr 10 zu sehen, welche denselben Gesamtquerschnitt wie die Eintrittsöffnung 11 des Auslaufrohres 10 aufweist und verstellbar entlang der Längsachse 2 angeordnet ist. Weiterhin ist im unteren Teil des Gehäuses 4 die Öffnung für die Fluidführung 33 zu sehen, welche entlang der Längsachse 2 angeordnet ist.

Das Auslaufrohr 10 weist eine Eintrittsöffnung 11 und eine Austrittsöffnung 12 sowie einen Radius *r*₂ 13 von der Längsachse 2 zur Außenwand des Auslaufrohres 10, eine Wanddicke d 14 und eine Düse zur Realisierung des Venturi-Effekts 17 auf. Eingezeichnet sind weiterhin für den oberen Teil der Reaktionskammer 18 die Deckfläche 20 und die Grundfläche 21 sowie der Übergangsbereich von der Deckfläche zur Grundfläche 22. Für den unteren Teil der Reaktionskammer 19 ist der Übergang der Grundfläche 24, der Sohlebereich 25, die untere Begrenzung 26 des Sohlebereichs 25 sowie der Abstand z 30 eingezeichnet.

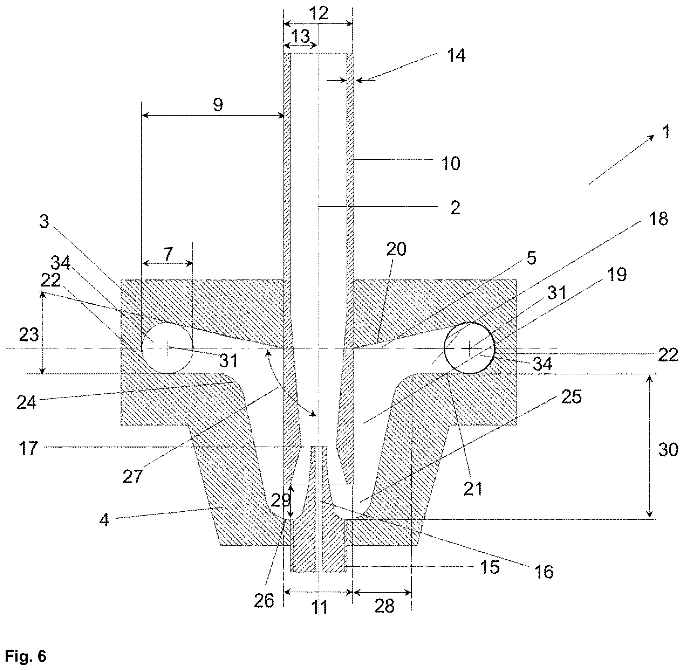

Figur 6 zeigt eine weitere vorteilhafte Ausführung der erfindungsgemäßen Reaktoranlage 1. Der Großteil der Merkmale des Aufbaus entspricht jenen aus der Draufsicht des Querschnitts aus Figur 1 bzw. 3, weshalb auf diese nicht weiter eingegangen wird. Der Längsschnitt der Reaktoranlage 1 verläuft so, dass der Fluideintrittsbereich 34 auf der in der Schnittdarstellung in Einbaulage linken und rechten Seite dargestellt ist. Das eingeleitete fluide Medium strömt auf der linken Seite aus der Schnittebene heraus. Bei dem Fluideintrittsbereich 34 auf der in der Schnittdarstellung rechten Seite wiederum strömt das eingeleitete fluide Medium in die Schnittebene hinein.

Der Anstellwinkel *α* 27 zur Längsachse 2 beträgt wiederum *α* = 90° und bezieht sich auf jenen Winkel, welcher sich, im Längsschnitt in Einbaulage gesehen, von der Mittelebene 5, die durch die Mittelpunkte 31 des Fluideintrittsbereiches 34 verläuft, zur Längsachse 2 einstellt. Der Abstand *b* 23 zwischen Deckfläche 20 und Grundfläche 21 ist im Bereich der Zuführungsöffnung (im Längsschnitt nicht gezeigt) und des Fluideintrittsbereiches 34 maximal (*b*ₘₐₓ) und entspricht dem kreisförmigen Durchmesser *d*_{z} 7 des Fluideintrittsbereiches 34. In Fließrichtung des fluiden Mediums verringert sich der Abstand *b* 23 zwischen Deckfläche 20 und Grundfläche 21 zur Außenwand des Auslaufrohres 10 hin, wodurch vorteilhaft eine zusätzliche Beschleunigung des fluiden Mediums erreicht wird.

Der benötigte Druck zur Erzeugung des Fluidwirbels und der Realisierung des Venturi-Effekts in der Düse 17 mit einem überlagerten Unterdruck von -0,99 bar liegt aufgrund der geringeren Fluidreibung in der Reaktionskammer 18, 19 mit 5,0 bar gegenüber der EP 1 294 474, welche bei gleichem Reaktionskammervolumen einen Druck von 6,0 bar benötigt, um ca. 17% niedriger.

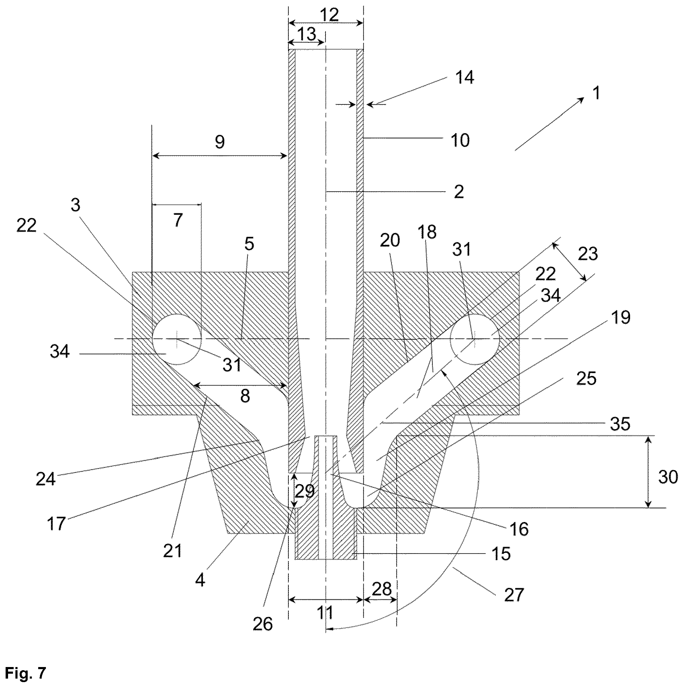

Figur 7 zeigt eine weitere vorteilhafte Ausführung der erfindungsgemäßen Reaktoranlage 1. Der Großteil der Merkmale des Aufbaus entspricht jenem aus der Draufsicht des Querschnitts Figur 1 bzw. 3, weshalb auf diese nicht weiter eingegangen wird. Der Längsschnitt der Reaktoranlage 1 verläuft so, dass der Fluideintrittsbereich 34 auf der in der Schnittdarstellung in Einbaulage linken und rechten Seite dargestellt ist. Das eingeleitete fluide Medium strömt auf der linken Seite aus der Schnittebene heraus. Bei dem Fluideintrittsbereich 34 auf der in der Schnittdarstellung rechten Seite wiederum strömt das eingeleitete fluide Medium in die Schnittebene hinein.

Der Abstand b 23 zwischen Deckfläche 20 und Grundfläche 21 ist konstant und entspricht dem kreisförmigen Durchmesser *d*_{z} 7 des Fluideintrittsbereiches 34. Der Anstellwinkel *α* 27 zur Längsachse 2 beträgt 110°. Der Anstellwinkel *α* 27 bezieht sich dabei auf jenen Winkel, welcher sich, im Längsschnitt in Einbaulage gesehen, von der fiktiven Zwischenebene 35, welche jeweils durch die Mittelpunkte 31 des Fluideintrittsbereiches 34 und parallel zu der Deckfläche 20 des oberen Teils der Reaktionskammer 18 verläuft, zur Längsachse 2 einstellt. Der Anstellwinkel *α* 27 = 110° bezieht sich dabei auf den sich in Einbaulage einstellenden Winkel unterhalb der fiktiven Zwischenebene 35, d.h. von der fiktiven Zwischenebene 35 zur Längsachse der Reaktionskammer 18, 19.

Der Radius *r*₁ 8 entspricht dem Abstand von der Grundfläche 21 des oberen Teils der Reaktionskammer 18 zur Außenwand des Auslaufrohres 10. Im Falle von *α* = 110° verringert sich *r*₁ 8 im oberen Teil der Reaktionskammer 18 kontinuierlich bis zum Übergang der Grundfläche 24 in den unteren Teil der Reaktionskammer 19.

Der benötigte Druck zur Erzeugung des Fluidwirbels und der Realisierung des Venturi-Effekts in der Düse 17 mit einem überlagerten Unterdruck von -0,99 bar liegt aufgrund der geringeren Fluidreibung in der Reaktionskammer 18, 19 mit 4,8 bar gegenüber der EP 1 294 474, welche bei gleichem Reaktionskammervolumen einen Druck von 6,0 bar benötigt, um ca. 20% niedriger.

### Bezugszeichen

- 1: Reaktoranlage
- 2: Längsachse der Reaktionskammer
- 3: Gehäuse, oberer Teil
- 4: Gehäuse, unterer Teil
- 5: Mittelebene
- 6: Zuführungsöffnung
- 7: Durchmesser *d*_{z} des Fluideintrittsbereiches, der sich an die zum oberen Teil der Reaktionskammer tangential angeordnete Zuführungsöffnung in Fließrichtung anschließt
- 8: Radius *r*₁ (Abstand von der Grundfläche des oberen Teils der Reaktionskammer zur Außenwand des Auslaufrohres)
- 9: Radius *r*ₘₐₓ (Abstand vom Übergangsbereich von der Deck- zur Grundfläche im oberen Teil der Reaktionskammer zur Außenwand des Auslaufrohres)
- 10: Auslaufrohr
- 11: Eintrittsöffnung des Auslaufrohres (Gesamtquerschnitt)
- 12: Austrittsöffnung des Auslaufrohres (Gesamtquerschnitt)
- 13: Radius *r*₂ des Auslaufrohres (von der Längsachse zur Außenwand)
- 14: Wanddicke d des Auslaufrohres
- 15: Fluidführung
- 16: Ausstülpung der Fluidführung
- 17: Düse zur Realisierung des Venturi-Effekts
- 18: Reaktionskammer, oberer Teil
- 19: Reaktionskammer, unterer Teil
- 20: Deckfläche des oberen Teils der Reaktionskammer
- 21: Grundfläche des oberen Teils der Reaktionskammer
- 22: Übergangsbereich von der Deck- zur Grundfläche im oberen Teil der Reaktionskammer
- 23: Abstand b zwischen der Deck- und der Grundfläche
- 24: Übergang der Grundfläche des unteren Teils der Reaktionskammer
- 25: Sohlebereich des unteren Teils der Reaktionskammer
- 26: untere Begrenzung des Sohlebereichs des unteren Teils der Reaktionskammer
- 27: Anstellwinkel *α* zur Längsachse
- 28: Radius *r*₃ (Abstand vom Übergang der Grundfläche des unteren Teils der Reaktionskammer zur Außenwand des Auslaufrohres)
- 29: Abstand *a* zwischen Eintrittsöffnung des Auslaufrohres und der unteren Begrenzung des unteren Teils der Reaktionskammer
- 30: Abstand *z* von der Grundfläche des unteren Teils der Reaktionskammer ab jener Stelle, an welcher die Deckfläche und die Grundfläche keinen konstanten oder abnehmenden Abstand mehr zueinander aufweisen zur unteren Begrenzung des Sohlebereichs des unteren Teils der Reaktionskammer
- 31: Mittelpunkt des Fluideintrittsbereiches
- 32: Öffnung im oberen Teil des Gehäuses für das Auslaufrohr
- 33: Öffnung im unteren Teil des Gehäuses für die Fluidführung
- 34: Fluideintrittsbereich
- 35: fiktive Zwischenebene

## Patentansprüche

1. Vorrichtung in Form einer strömungsdynamischen Reaktoranlage (1) zur Aufnahme eines fluiden Mediums zur Erzeugung von mindestens einem geführten Fluidwirbel, umfassend ein Gehäuse (3, 4) und ein Auslaufrohr (10), wobei
- das Gehäuse (3, 4)
∘ mittels derfluidkontaktseitigen Innenwände einen um eine Längsachse (2) rotationssymmetrischen fluidführenden Hohlraum bildet, im Folgenden Reaktionskammer (18, 19) genannt,
∘ wobei die Reaktionskammer (18, 19) in einen oberen (18) und einen unteren Teil (19) aufgeteilt ist und der obere Teil (3) des Gehäuse den oberen Teil (18) der Reaktionskammer und der untere Teil (4) des Gehäuses den unteren Teil (19) der Reaktionskammer bildet und
▪ der obere Teil der Reaktionskammer (18)
• eine Deckfläche (20) und eine Grundfläche (21) aufweist, wobei die Deck- (20) bzw. Grundfläche (21) einen Anstellwinkel (27) zur Längsachse von 80° bis 115° besitzen,
• einen Übergangsbereich (22) von der Deck- zur Grundfläche aufweist,
• im Übergangsbereich (22) von der Deck- zur Grundfläche einen maximalen Radius (9) bezogen auf die Außenwand des Auslaufrohres (10) aufweist,
• im Übergangsbereich (22) von der Deckfläche zur Grundfläche mindestens eine tangential zur Mantelfläche des oberen Teils der Reaktionskammer (18) angeordnete Zuführungsöffnung (6) mit einen sich in Fließrichtung anschließenden Fluideintrittsbereich (34) aufweist, und
▪ der untere Teil der Reaktionskammer (19) sich in einem Abstand z (30) von einem Übergang (24) der Grundfläche (21) bis zu der unteren Begrenzung eines gekrümmten Sohlebereich (25) erstreckt, wo eine geometrisch aufsteigend geformte Fluidführung (15) ausgebildet ist, das fluide Medium in eine Eintrittsöffnung (1 1) des Auslaufrohres (10) umzulenken, und
- das Auslaufrohr (10) in seiner Längsachse mit der Längsachse der rotationssymmetrischen Reaktionskammer (18, 19) zusammenfällt und sich durch den oberen Teil (3) des Gehäuses erstreckt und die Eintrittsöffnung (11) des Auslaufrohres (10) in einem Abstand a (29) zur unteren Begrenzung (26) des gekrümmten Sohlebereichs (25) angeordnet ist **dadurch gekennzeichnet, dass** ein Abstand (23) zwischen Deckfläche (20) und Grundfläche (21) vom Übergangsbereich (22) der Deckfläche (20) zur Grundfläche (21) bis zum Übergang (24) der Grundfläche (21) in den unteren Teil der Reaktionskammer, konstant ausgebildet ist und dem Durchmesser dz des Fluideintrittsbereiches entspricht oder dass ein Abstand (23) zwischen Deckfläche (20) und Grundfläche (21) von dem Übergangsbereich (22) der Deckfläche (20) zur Grundfläche (21) bis zu dem Übergang (24) der Grundfläche (21) in den unteren Teil der Reaktionskammer, also in Richtung Auslaufrohr, kontinuierlich abnehmend ausgebildet ist, wobei der Abstand der Deck- zur Grundfläche bei der mindestens einen Zuführungsöffnung und dem sich anschließenden Fluideintrittsbereich maximal ist und dem Durchmesser d_{z} des Fluideintrittsbereiches entspricht.

2. Vorrichtung nach Anspruch 1, **dadurch gekennzeichnet, dass** das Auslaufrohr (10) im mündungsnahen Bereich, der sich an die Eintrittsöffnung (11) anschließt, als Düse zur Realisierung eines Venturi-Efiekts (17) ausgebildet ist, wobei die Düse zur Realisierung des Venturi-Effekts (17) als Venturi- oder Lavaldüse ausgestaltet ist und eine Ausstülpung (16) der Fluidführung (15) in der Düse zur Realisierung des Venturi-Effekts (17) endet.

3. Vorrichtung nach Anspruch 1 bis 2, **dadurch gekennzeichnet, dass** der Abstand z (30) mindestens die Hälfte des Durchmessers des Fluideintrittsbereiches (7) beträgt, nämlich z ≥ ½ d_{z}.

4. Vorrichtung nach Anspruch 1 bis 3, **dadurch gekennzeichnet, dass** ein Radius r₁ (8) jenen Abstand definiert, der von der Grundfläche (21) des oberen Teils der Reaktionskammer (18) zur Außenwand des Auslaufrohres (10) entlang einer Ebene parallel zu einer Mittelebene (5) verläuft, und ein Radius *r*₃ (28) jenen Abstand definiert, der sich vom Beginn des Übergangs der Grundfläche (24) des unteren Teils der Reaktionskammer (19) bis zu einer Außenwand des Auslaufrohres (10) erstreckt, wobei r₁ (8) mindestens größer als die Summe des Durchmessers des Fluideintrittsbereiches d_{z} (7) und des Abstands *r*₃ (28), nämlich (r1 ≥ d_{z}.+ r₃) ist.

5. Vorrichtung nach Anspruch 1 bis 4, **dadurch gekennzeichnet, dass** der obere Teil der Reaktionskammer (18) mehr als eine tangential zur Mantelfläche des oberen Teils der Reaktionskammer (18) angeordnete Zuführungsöffnung (6) aufweist.

6. Vorrichtung nach Anspruch 1 bis 5, **dadurch gekennzeichnet, dass** das Auslaufrohr (10) und/oder die Fluidführung (15) entlang der Längsachse (2) verstell- und verschiebbar ist.

7. Vorrichtung nach Anspruch 1 bis 6, **dadurch gekennzeichnet, dass** die Fluidführung (15) entlang der Längsachse (2) eine Durchgangsbohrung aufweist.

8. Verfahren zum Betrieb einer Vorrichtung nach Anspruch 1 bis 7 wobei das zu behandelnde fluide Medium in mindestens einem Volumenstrom über die mindestens eine Zuführungsöffnung (6) in den oberen Teil der rotationssymmetrischen Reaktionskammer (18) des Gehäuses (3, 4) eingeleitet wird, wobei der mindestens eine Volumenstrom in eine in Fließrichtung zum unteren Teil der Reaktionskammer (19) gerichteten Translations- und Rotationsbewegung entlang der Längsachse (2) versetzt wird und der mindestens eine Volumenstrom einen rotierenden Fluidwirbel bildet, welcher strömungstechnisch eine turbulente Grenzschicht formtund hohe Zentrifugalkräfte in dem Fluidwirbel bereitgestellt werden und dass der Fluidwirbel an der Fluidführung (15) in eine der in Einbaulage nach unten gerichteten Translations- und Rotationsbewegung entgegengesetzte aufsteigende Bewegung entlang der Längsachse (2) in die Eintrittsöffnung (11) des Auslaufrohres (10) umgelenkt wird,
**dadurch gekennzeichnet,**
**dass** durch die strömungsdynamische Behandlung des mindestens einen Fluidwirbels in der Vorrichtung nach einem der Ansprüche 1 bis 9 die Umsetzung und/oder mechanische und physikalische Zerstörung und/oder Radikalisierung von im fluiden Medium befindlichen chemischen Stoffen oder Mikroorganismen stattfindet.

9. Verfahren nach Anspruch 8, **dadurch gekennzeichnet, dass** durch die Durchgangsbohrung entlang der Längsachse (2) in der Fluidführung (15) mindestens ein zusätzliches fluides Medium in die Eintrittsöffnung (11) des Auslaufrohres (10) ansaugbar ist, wobei es dasselbe und/oder ein anderes zusätzliches fluides Medium ist als das in der mindestens einen Zuführungsöffnung (6) eingeleitete fluide Medium.

10. Verwendung einer Vorrichtung nach Anspruch 1 bis 7 zur Vorbehandlung, Aufbereitung, Entkeimung, Desinfektion und/oder Initiierung chemischer Reaktionen von und in fluiden Medien.

11. Verwendung einer Vorrichtung nach Anspruch 1 bis 7 zur Durchführung des Verfahrens nach Anspruch 8 oder 9.

## Claims

1. A device in the form of a flow dynamic reactor facility (1) for receiving a fluid medium for producing at least one guided fluid eddy, including a housing (3, 4) and an outlet pipe (10), wherein
- the housing (3, 4)
∘ by means of the fluid-contact-side inner walls forms a fluid-carrying hollow chamber, hereinafter called reaction chamber (18, 19), that is rotationally symmetrical about a longitudinal axis (2);
∘ wherein the reaction chamber (18, 19) is split into an upper part (18) and a lower
part (19) and the upper part (3) of the housing forms the upper part (18) of the reaction chamber and the lower part (4) of the housing forms and the lower part (4) of the housing forms the lower part (19) of the reaction chamber, and
▪ the upper part of the reaction chamber (18)
• has a top face (20) and a bottom face (21), wherein the top face (20) and/or bottom face (21) have a setting angle (27) to the longitudinal axis of 80° to 115°,
• has a transition region (22) from the top face to the bottom face,
• in the transition region (22) from the top face to the bottom face has a maximum radius (9) referred to the outer wall of the outlet pipe (10),
• in the transition region (22) from the top face to the bottom face has at least one delivery opening (6), located tangentially to the jacket face of the upper part of the reaction chamber (18), with a fluid inlet region (34) adjoining it in the flow direction; and
▪ the lower part of the reaction chamber (19) extends at a spacing z (30) from the transition (24) from the bottom face (21) to the lower boundary of a curved floor region (25), where a geometrically ascending-shaped fluid passage (15) is embodied, in order to divert the fluid medium into an inlet opening (11) of the outlet pipe (10); and
- the outlet pipe (10) coincides in its longitudinal axis with the longitudinal axis of the rotationally symmetrical reaction chamber (18, 19) and extends through the upper part (3) of the housing, and the inlet opening (11) of the outlet pipe (10) is located at a spacing a (29) from the lower boundary (26) of the curved floor region (25),
**characterized in that**
a spacing (23) between the top face (20) and bottom face (21) from the transition region (22) of the top face (20) to the bottom face (21) is embodied as constant as far as the transition (24) from the bottom face (21) to the lower part of the reaction chamber and is equivalent to the diameter d_{z} of the fluid inlet region, or
that a spacing (23) between the top face (20) and bottom face (21) from the transition region (22) of the top face (20) to the bottom face (21) into the lower part of the reaction chamber, that is, in the direction of the outlet pipe, is embodied as decreasing continuously, and the spacing of the top face to the bottom face is at a maximum at the at least one delivery opening and the adjoining fluid inlet region and is equivalent to the diameter d_{z} of the fluid inlet region.

2. The device of claim 1, **characterized in that** the outlet pipe (10), in the region near the mouth that adjoins the inlet opening (11), is embodied as a nozzle for producing a Venturi effect (17), and the nozzle for attaining the Venturi effect (17) is designed as a Venturi or a Laval nozzle, and an eversion (16) of the fluid passage (15) ends in the nozzle for attaining the Venturi effect (17).

3. The device of claims 1 through 2, **characterized in that** the spacing z (30) amounts to at least half of the diameter of the fluid inlet region (7), namely d_{z} (z≥ ½ d_{z}).

4. The device of claims 1 through 3, **characterized in that** a radius r₁ (8) defines the spacing which extends from the bottom face (21) of the upper part of the reaction chamber (18) to the outer wall of the outlet pipe (10) along a plane parallel to a center plane (5), and a radius r₃ (28) defines the spacing that extends from the beginning of the transition of the bottom face (24) of the lower part of the reaction chamber (19) to an outer wall of the outlet pipe (10), where r₁ (8) is at least greater than the sum of the diameter of the fluid inlet region d_{z} (7) and of the spacing r₃ (28), namely (n≥d_{z} + r₃).

5. The device of claims 1 through 4, **characterized in that** the upper part of the reaction chamber (18) has more than one delivery opening (6) located tangentially to the jacket face of the upper part of the reaction chamber (18).

6. The device of claims 1 through 5, **characterized in that** the outlet pipe (10) and/or the fluid passage (15) is adjustable and shiftable along the longitudinal axis (2).

7. The device of claims 1 through 6, **characterized in that** the fluid passage (15) has a through bore along the longitudinal axis (2).

8. A method for operating a device of claims 1 through 7, wherein the fluid medium to be treated is guided in at least one volume flow via the at least one delivery opening (6) into the upper part of the rotationally symmetrical reaction chamber (18) of the housing (3, 4), wherein the at least one volume flow is set into a translational and rotary motion along the longitudinal axis (2), which motion is oriented in the flow direction relative to the lower part of the reaction chamber (19), and the at least one volume flow forms a rotating fluid eddy, which in terms of flow technology forms a turbulent boundary layer and high centrifugal forces in the fluid eddy arise; and that the fluid eddy is diverted at the fluid passage (15) into the inlet opening (11) of the outlet pipe (10) in what in the installed state is an ascending motion that is opposed to a downward-oriented translational and rotary motion, **characterized in that** as a result of the flow dynamics treatment of the at least one fluid eddy in the device of one of claims 1 through 9, the conversion and/or mechanical and physical destruction and/or radicalization of chemical substances or microorganism found in the fluid medium occurs.

9. The method of claim 8, **characterized in that** through the through bore along the longitudinal axis (2) in the fluid passage (15), at least one additional fluid medium can be aspirated into the inlet opening (11) of the outlet pipe (10), and it is the same and/or a different additional fluid medium from the fluid medium introduced into the at least one delivery opening (6).

10. Usage of a device of claims 1 through 7 for pretreatment, processing, sterilization, disinfection, and/or initiation of chemical reactions of and in fluid media.

11. Usage of a device of claims 1 through 7, for performing the method of claim 8 or 9.

## Revendications

1. Dispositif sous forme d'une installation de réacteur à écoulement dynamique (1) pour l'inclusion d'un support fluide pour la production au minimum d'un tourbillon fluide guidé comportant un habitacle (3,4) et un tuyau d'évacuation (10) où l'habitacle (3,4) forme par le biais de parois intérieures à côté du contact fluide une cavité à rotation symétrique menant le fluide tout au long de l'axe longitudinal (2) décrit ci-après comme étant la chambre de réaction (18,19)
Où la chambre de réaction (18,19) est répartie en parti supérieure (18) et parti inférieure (19) et la parti supérieure (3) de l'habitacle forme la parti supérieure (18) de la chambre de réaction et la parti inférieure (4) de l'habitacle forme la parti inférieure (19) de la chambre de réaction
Et est répartie en une parti supérieure (18) et en une parti inférieure (19) et la parti supérieure de la chambre de réaction (18) présente une surface de recouvrement (20) et une surface de base (21) où la surface de recouvrement (20) ou le cas échéant la surface de base (21) possèdent un angle d'attaque à l'axe longitudinal allant de 80° jusqu'à 115°
Présente une zone de transition (22) de la surface de recouvrement vers la surface de base (22)
Présente un périmètre maximal (9) en relation avec la paroi externe du tuyau d'évacuation (10) dans la zone de transition de la surface de recouvrement vers la surface de base
Présente au minimum une ouverture d'alimentation (6) disposée tangentiellement vers la surface extérieure de la parti supérieure de la chambre de réaction (18) dans la zone de transition de la surface de recouvrement vers la surface de base avec une zone d'entrée du fluide à direction d'écoulement ultérieure
Et la parti inférieure de la chambre de réaction (19) s'étend en une distance (30) de la transition de la surface de base (21) jusqu'à la limitation inférieure de la zone sommelière courbée (25) où un écoulement de fluide géométrique croissant (15) est formé pour dévier le support fluide vers une ouverture d'entrée (11) du tuyau d'évacuation (10)
Le tuyau d'évacuation (10) coïncide dans son axe longitudinal avec l'axe longitudinal de la chambre de réaction à rotation symétrique (18,19) et s'étend à travers la parti supérieure (3) de l'habitacle, tandis que l'ouverture d'entrée (11) du tuyau d'évacuation (10) est disposée en distance a (29) vers la limitation inférieure (26) de la zone sommelière courbée (25)
Caractérisé de sorte qu'une distance (23) est formée constamment entre la surface de recouvrement (20) et la surface de base (21) de la zone de transition (22) de la surface de recouvrement (20) vers la surface de base (21) jusqu'à la transition (24) de la surface de base (21) dans la parti inférieure de la chambre de réaction donc dans la direction du tuyau d'évacuation correspondant à un diamètre dz de la zone d'entrée du fluide ou une distance (23) entre la surface de recouvrement (20) et la surface de base (21) est formée à diminution continue dans la zone de transition (22) de la surface de recouvrement (20) vers la surface de base (21) jusqu'à la transition (24) de la surface de base (21) dans la parti inférieure de la chambre de réaction donc en direction du tuyau d'évacuation où la distance de la surface de recouvrement vers la surface de base est au maximum et au minimum au niveau de l'ouverture d'alimentation et de la zone d'entrée du fluide ultérieure et correspond au diamètre dz de la zone d'entrée du fluide.

2. Dispositif selon la revendication 1 est caractérisé de sorte que le tuyau d'évacuation (10) est formé d'une tuyère dans la zone à proximité de l'embouchure découlant vers l'ouverture d'entrée (11) pour la réalisation de l'effet Venturi (17) où la tuyère pour la réalisation de l'effet Venturi (17) est conçue en qualité de tuyère Venturi ou Laval et une protubérance (16) de la conduite du fluide (15) finit dans la tuyère pour la réalisation de l'effet Venturi (17).

3. Dispositif selon la revendication 1 jusqu'à 2 est caractérisé de sorte que la distance z (30) se situe au minimum en la moitié du diamètre de la zone d'entrée du fluide (7) en l'occurrence z≥1/2dz.

4. Dispositif selon la revendication 1 jusqu'à 3 est caractérisé de sorte que le périmètre r1 (8) définit la distance s'étendant de la surface de base (21) de la parti supérieure de la chambre de réaction (18) vers la paroi externe du tuyau d'évacuation (10) au long du niveau parallèle au niveau médiale (5) et un périmètre r3 (23) définit la distance s'étendant du début de la transition de la surface de base (24) de la parti inférieure de la chambre de réaction (19) jusqu'à la paroi externe du tuyau d'évacuation (10) où r1 (8) est plus grand au minimum que la somme du diamètre de la zone d'entrée du fluide dz (7) et de la distance r3 (28) en l'occurrence (r1≥dz.+r3).

5. Dispositif selon la revendication 1 jusqu'à 4 est caractérisé de sorte que la parti supérieure de la chambre de réaction (18) présente plus qu'une ouverture d'alimentation disposée tangentiellement vers une surface extérieure de la parti supérieure de la chambre de réaction (18).

6. Dispositif selon la revendication 1 jusqu'à 5 est caractérisé de sorte que le tuyau d'évacuation (10) et/ou la conduite du fluide (15) tout au long de l'axe longitudinal (2) est réglable et déplaçable.

7. Dispositif selon la revendication 1 jusqu'à 6 est caractérisé de sorte que la conduite du fluide (15) présente tout au long de l'axe longitudinal (2) un alésage traversant.

8. Dispositif selon la revendication 1 jusqu'à 7 où le support fluide traité est introduit en au minimum un courant volumineux via au minimum une ouverture d'alimentation (6) dans la parti supérieure de la chambre de réaction à rotation symétrique (18) de l'habitacle (3,4) où au minimum le courant volumineux est déplacé en un mouvement de translation et de rotation orienté vers une direction d'écoulement vers la parti inférieure de la chambre de réaction (19) tout au long de l'axe longitudinal (2) et que le courant volumineux forme au minimum un tourbillon fluide rotatif formant une couche limite turbulente à technique d'écoulement et des forces centrifugées sont fournies dans l'écoulement fluide et que l'écoulement fluide est dévié en un mouvement de translation et de rotation orienté vers le bas dans sa position de montage opposée à un mouvement croissant tout au long de l'axe longitudinal (2) dans l'ouverture d'entrée (11) du tuyau d'évacuation (10) est caractérisé de sorte que l'application et/ou la destruction mécanique et physique et/ou la radicalisation des matières chimiques se trouvant dans le support fluide ou dans les microorganismes ont lieu par le traitement à courant dynamique du tourbillon fluide au minimum dans le dispositif selon la revendication 1 jusqu'à 9.

9. Dispositif selon la revendication 1 jusqu'à 8 est caractérisé de sorte qu'au minimum un support fluide supplémentaire est aspirable par l'alésage traversant tout au long de l'axe longitudinal (2) dans la conduite du fluide (15) dans l'ouverture du fluide (11) du tuyau d'évacuation (10) où le même support fluide supplémentaire et/ou un autre support fluide supplémentaire que le support fluide est introduit au minimum dans l'ouverture d'alimentation.

10. Utilisation d'un dispositif selon la revendication 1 jusqu'à 9 pour le prétraitement, la préparation, la stérilisation, la désinfection et/ou l'initiation de réactions chimiques de et dans les supports fluides.

11. Utilisation d'un dispositif selon la revendication 1 jusqu'à 9 pour la mise en œuvre du procédé selon la revendication 8 ou 9.
